(19) Europäisches Patentamt
European Patent Office
Office européen des brevets

(11) Veröffentlichungsnummer: **0 302 378**
**A2**

# (12) EUROPÄISCHE PATENTANMELDUNG

(21) Anmeldenummer: 88112129.7

(22) Anmeldetag: 27.07.88

(51) Int. Cl.4: C07D 239/47 , C07D 239/42 , C07D 239/52 , C07D 403/12 , C07D 409/12 , C07D 251/46 , A01N 47/44

(30) Priorität: 07.08.87 DE 3726269

(43) Veröffentlichungstag der Anmeldung: 08.02.89 Patentblatt 89/06

(84) Benannte Vertragsstaaten: BE CH DE ES FR GB IT LI NL

(71) Anmelder: BAYER AG
D-5090 Leverkusen 1, Bayerwerk(DE)

(72) Erfinder: Müller, Klaus-Helmut, Dr.
Bockhackstrasse 55
D-4000 Düsseldorf 13(DE)
Erfinder: Fest, Christa, Dr.
Im Johannistal 20
D-5600 Wuppertal 1(DE)
Erfinder: Kirsten, Rolf, Dr.
Carl-Langhans-Strasse 27
D-4019 Monheim(DE)
Erfinder: Pfister, Theodor, Dr.
Lichtenberger Strasse 30
D-4019 Monheim(DE)
Erfinder: Riebel, Hans-Jochem, Dr.
In der Beek 92
D-5600 Wuppertal 1(DE)
Erfinder: Santel, Hans-Joachim, Dr.
Grünstrasse 9a
D-5090 Leverkusen 1(DE)
Erfinder: Schmidt, Robert R., Dr.
Im Waldwinkel 110
D-5060 Bergisch Gladbach 2(DE)
Erfinder: Strang, Harry, Dr.
Unterdorfstrasse 6A
D-4000 Düsseldorf 31(DE)

(54) **Sulfonylaminoguanidinoazine.**

(57) Die Erfindung betrifft neue Sulfonylaminoguanidinoazine der allgemeinen Formel (I)

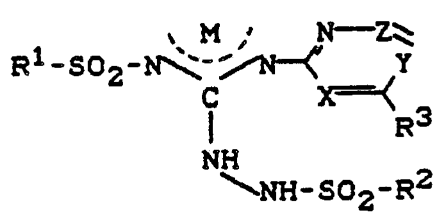

(I)

EP 0 302 378 A2

(in welcher $R^1$, $R^2$, $R^3$, X, Y, Z und M die in der Beschreibung angegebenen Bedeutungen haben) sowie ein Verfahren zu ihrer Herstellung und ihre Verwendung als Herbizide.

## Sulfonylaminoguanidinoazine

Die Erfindung betrifft neue Sulfonylaminoguanidinoazine, Verfahren zu ihrer Herstellung und ihre Verwendung als Herbizide.

Es ist bereits bekannt, daß bestimmte Aminoguanidinoazine, wie z. B. N′-(4,6-Dimethylpyrimidin-2yl)-N″-acetylamino-N‴-(2-chlorophenylsulfonyl)-guanidin, herbizide Eigenschaften aufweisen (vgl. EP-A 121 082). Die herbizide Wirkung der bisher bekannten Aminoguanidinoazine ist jedoch nicht immer ganz zufriedenstellend.

Es wurden nun neue Sulfonylaminoguanidinoazine der allgemeinen Formel (I)

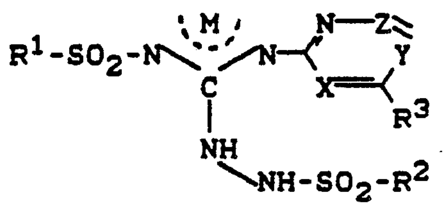

(I)

in welcher

$R^1$ für einen gegebenenfalls substituierten Rest aus der Reihe Alkyl, Aralkyl, Aryl und Hetaryl steht,

$R^2$ für einen gegebenenfalls substituierten Rest aus der Reihe Alkyl, Aralkyl, Aryl, Hetaryl und Dialkylamino steht,

$R^3$ für Wasserstoff, Halogen, Hydroxy, Alkyl, Halogenalkyl, Alkoxyalkyl, Alkoxy, Halogenalkoxy, Alkylthio, Halogenalkylthio, Amino, Alkylamino oder Dialkylamino steht,

X für Stickstoff oder eine -CH-Gruppierung steht,

Y für Stickstoff oder eine $-CR^4$-Gruppierung steht, worin

$R^4$ für Wasserstoff, Halogen, Cyano, Alkyl, Formyl, Alkyl-carbonyl oder Alkoxy-carbonyl steht,

Z für Stickstoff oder eine $-CR^5$-Gruppierung steht, worin

$R^5$ für Wasserstoff, Halogen, Hydroxy, Alkyl, Alkoxy, Halogenalkoxy, Alkythio, Alkylamino oder Dialkylamino steht, und

M für Wasserstoff, ein Metalläquivalent, ein Ammonium-, ein Alkylammonium-, ein Dialkylammonium-oder Trialkylammoniumäquivalent steht,

gefunden, wobei N′-(4,6-Dimethyl-pyrimidin-2-yl)-N″-(4-methyl-phenylsulfonylamino)-N‴-(2-chlor-phenylsulfonyl)-guanidin - bekannt aus EP-A 121 082 - ausgenommen ist.

Die allgemeine Formel (I) steht - wenn M für Wasserstoff steht - für die einzelnen möglichen Tautomeren der Formeln (IA), (IB) und (IC)

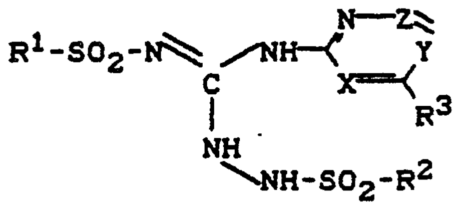

(IA)

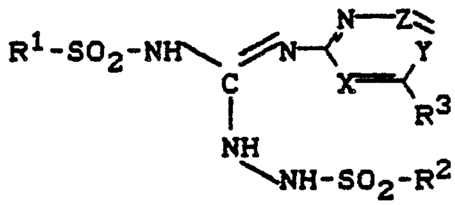

(IB)

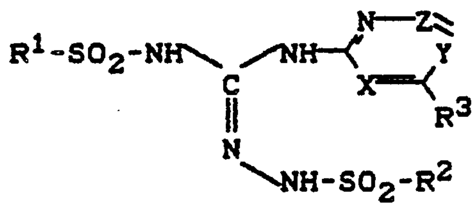

(IC)

sowie für Gemische dieser Tautomeren.

Man erhält die neuen Sulfonylaminoguanidinoazine der allgemeinen Formel (I), wenn man Sulfonylverbindungen der allgemeinen Formel (II)

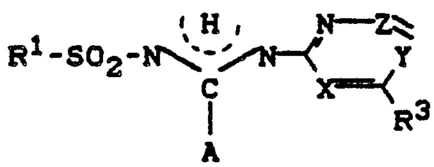

(II)

in welcher
$R^1$, $R^3$, X, Y und Z die oben angegebenen Bedeutungen haben und
A für eine der nachstehend angegebenen Abgangsgruppen $R^6-SO_2-N-OR^7$

oder $-Q-R^8$ steht, worin
$R^6$ die oben für $R^1$ angegebene Bedeutung hat, aber nicht in jedem Einzelfall mit $R^1$ identisch sein muß,
$R^7$ für Alkyl, Alkenyl oder Aralkyl steht,
$R^8$ für Alkyl, Aralkyl oder Aryl steht und
Q für Sauerstoff oder Schwefel steht,
mit Sulfonsäurehydraziden der allgemeinen Formel (III)

$R^2 - SO_2 - NH - NH_2$ (III)

in welcher
$R^2$ die oben angegebene Bedeutung hat,
gegebenenfalls in Gegenwart eines Verdünnungsmittels umsetzt und gegebenenfalls die so erhaltenen Produkte nach üblichen Methoden in Salze überführt.

Die neuen Sulfonylaminoguanidinoazine der allgemeinen Formel (I) zeichnen sich durch starke herbizide Wirksamkeit aus.

Überraschenderweise zeigen die neuen Verbindungen der allgemeinen Formel (I) erheblich bessere herbizide Wirkung als vorbekannte Aminoguanidinoazine gleicher Wirkungsrichtung.

Gegenstand der Erfindung sind vorzugsweise Verbindungen der Formel (I), in welcher
$R^1$ für den Rest

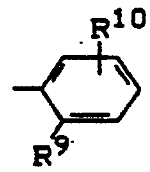

steht, worin

$R^9$ und $R^{10}$ gleich oder verschieden sind und für Wasserstoff, Fluor, Chlor, Brom, Iod, Cyano, Nitro, $C_1$-$C_6$-Alkyl [welches gegebenenfalls durch Fluor, Chlor, Brom, Cyano, Carboxy, $C_1$-$C_4$-Alkoxycarbonyl, $C_1$-$C_4$-Alkylamino-carbonyl, Di-($C_1$-$C_4$-alkyl)-amino-carbonyl, Hydroxy, $C_1$-$C_4$-Alkoxy, For myloxy, $C_1$-$C_4$-Alkylcarbonyloxy, $C_1$-$C_4$-Alkoxy-carbonyloxy, $C_1$-$C_4$-Alkylamino-carbonyloxy, $C_1$-$C_4$-Alkylthio, $C_1$-$C_4$-Alkylsulfinyl, $C_1$-$C_4$-Alkylsulfonyl, Di-($C_1$-$C_4$-alkyl)-aminosulfonyl, $C_3$-$C_6$-Cycloalkyl oder Phenyl substituiert ist], für $C_2$-$C_6$-Alkenyl [welches gegebenenfalls durch Fluor, Chlor, Brom, Cyano, $C_1$-$C_4$-Alkoxy-carbonyl, Carboxy oder Phenyl substituiert ist], für $C_2$-$C_6$-Alkinyl [welches gegebenenfalls durch Fluor, Chlor, Brom, Cyano, $C_1$-$C_4$-Alkoxy-carbonyl, Carboxy oder Phenyl substituiert ist], für $C_1$-$C_4$-Alkoxy [welches gegebenenfalls durch Fluor, Chlor, Brom, Cyano, Carboxy, $C_1$-$C_4$-Alkoxy-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für $C_1$-$C_4$-Alkylthio [welches gegebenenfalls durch Fluor, Chlor, Brom, Cyano, Carboxy, $C_1$-$C_4$-Alkoxy-carbonyl, $C_1$-$C_4$-Alkylthio, $C_1$-$C_4$-Alkylsulfinyl oder $C_1$-$C_4$-Alkylsulfonyl substituiert ist], für $C_3$-$C_6$-Alkenyloxy [welches gegebenenfalls durch Fluor, Chlor, Brom, Cyano oder $C_1$-$C_4$-Alkoxy-carbonyl substituiert ist], für $C_2$-$C_6$-Alkenylthio [welches gegebenenfalls durch Fluor, Chlor, Brom, Cyano, Nitro, $C_1$-$C_3$-Alkylthio oder $C_1$-$C_4$-Alkoxycarbonyl substituiert ist], $C_3$-$C_6$-Alkinyloxy, $C_3$- $C_6$-Alkinylthio oder für den Rest $-S(O)_p-R^{11}$ stehen, wobei

p für die Zahlen 1 oder 2 steht und

$R^{11}$ für $C_1$-$C_4$-Alkyl [welches gegebenenfalls durch Fluor, Chlor, Brom, Cyano oder $C_1$-$C_4$-Alkoxy-carbonyl substituiert ist], $C_3$-$C_6$-Alkenyl, $C_3$-$C_6$-Alkinyl, $C_1$-$C_4$-Alkoxy, $C_1$-$C_4$-Alkoxy-$C_1$-$C_4$-alkylamino, $C_1$-$C_4$-Alkylamino, Di-($C_1$-$C_4$--alkyl)-amino oder für den Rest $-NHOR^{12}$ steht, wobei

$R^{12}$ für $C_1$-$C_{12}$-Alkyl [welches gegebenenfalls durch Fluor, Chlor, Cyano, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkyl-carbonyl, $C_1$-$C_4$-Alkoxy-carbonyl, $C_1$-$C_4$-Alkylamino-carbonyl oder Di-($C_1$-$C_4$-alkyl)-amino-carbonyl substituiert ist], für $C_3$-$C_6$-Alkenyl [welches gegebenenfalls durch Fluor, Chlor oder Brom substituiert ist], $C_3$-$C_6$-Alkinyl, $C_3$-$C_6$-Cycloalkyl, $C_3$-$C_6$-Cycloalkyl-$C_1$-$C_2$-alkyl, Phenyl-$C_1$-$C_2$-alkyl [welches gegebenenfalls durch Fluor, Chlor, Nitro, Cyano, $C_1$-$C_4$-Alkyl, $C_1$-$C_4$-Alkoxy oder $C_1$- $C_4$-Alkoxy-carbonyl substituiert ist], für Benzhydryl oder für Phenyl [welches gegebenenfalls durch Fluor, Chlor, Nitro, Cyano, $C_1$-$C_4$-Alkyl, Trifluormethyl, $C_1$-$C_4$-Alkoxy, $C_1$-$C_2$-Fluoralkoxy, $C_1$-$C_4$-Alkythio, Trifluormethylthio oder $C_1$-$C_4$-Alkoxy-carbonyl substituiert ist] steht,

$R^9$ und $R^{10}$ weiterhin für Phenyl oder Phenoxy, für Amino, $C_1$-$C_4$-Alkylcarbonylamino $C_1$-$C_4$-Alkoxy-carbonylamino, $C_1$-$C_4$-Alkylamino-carbonyl-amino, Di-($C_1$-$C_4$-alkyl)-amino-carbonylamino, oder für den Rest $-CO-R^{13}$ stehen, wobei

$R^{13}$ für $C_1$-$C_6$-Alkyl, $C_1$-$C_6$-Alkoxy, $C_3$-$C_6$-Cycloalkoxy, $C_3$-$C_6$-Alkenyloxy, $C_1$-$C_4$-Alkylthio, $C_1$-$C_4$-Alkylamino, $C_1$-$C_4$-Alkoxyamino, $C_1$-$C_4$-Alkoxy-$C_1$-$C_4$-alkyl-amino oder Di-($C_1$-$C_4$-alkyl)-amino steht [welche gegebenenfalls durch Fluor und/oder Chlor substituiert sind],

$R^9$ und $R^{10}$ weiterhin für $C_1$-$C_4$-Alkylsulfonyloxy, Di-($C_1$-$C_4$-alkyl)-aminosulfonylamino, Thiazolyloxy oder für den Rest $-CH=N-R^{14}$ stehen, wobei

$R^{14}$ für gegebenenfalls durch Fluor, Chlor, Cyano, Carboxy, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes $C_1$-$C_6$-Alkyl, für gegebenenfalls durch Fluor oder Chlor substituiertes Benzyl, für gegebenenfalls durch Fluor oder Chlor substituiertes $C_3$-$C_6$-Alkenyl oder $C_3$-$C_6$-Alkinyl, für gegebenenfalls durch Fluor, Chlor, Brom, $C_1$-$C_4$-Alkyl, $C_1$-$C_4$-Alkoxy, Trifluormethyl, Trifluormethoxy oder Trifluormethylthio substituiertes Phenyl, für gegebenenfalls durch Fluor und/oder Chlor substituiertes $C_1$-$C_6$-Alkoxy, $C_3$-$C_6$-Alkenoxy, $C_3$-$C_6$-Alkinoxy oder Benzyloxy, für Amino, $C_1$-$C_4$-Alkylamino, Di-($C_1$-$C_4$-alkyl)-amino, Phenylamino, $C_1$-$C_4$-Alkyl-carbonyl-amino, $C_1$-$C_4$-Alkoxy-carbonylamino, $C_1$-$C_4$-Alkyl-sulfonylamino oder für gegebenenfalls durch Fluor, Chlor, Brom oder Methyl substituiertes Phenylsulfonylamino steht,

worin weiter

$R^1$ für den Rest

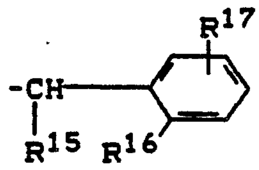

steht, worin
$R^{15}$ für Wasserstoff oder $C_1$-$C_4$-Alkyl steht,
$R^{16}$ und $R^{17}$ gleich oder verschieden sind und für Wasserstoff, Fluor, Chlor, Brom, Nitro, Cyano, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], Carboxy, $C_1$-$C_4$-Alkoxy-carbonyl, $C_1$-$C_4$-Alkylsulfonyl oder Di-$C_1$-$C_4$-alkyl)-aminosulfonyl stehen; worin weiter
$R^1$ für den Rest

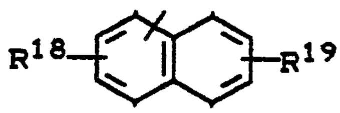

steht, worin
$R^{18}$ und $R^{19}$ gleich oder verschieden sind und für Wasserstoff, Fluor, Chlor, Brom, Nitro, Cyano, $C_1$-$C_4$-Alkyl [welches gegebenenfalls durch Fluor und/oder Chlor substituiert ist] oder $C_1$-$C_4$-Alkoxy [welches gegebenenfalls durch Fluor und/oder Chlor substituiert ist], stehen; worin weiter
$R^1$ für den Rest

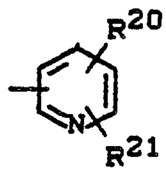

steht, worin
$R^{20}$ und $R^{21}$ gleich oder verschieden sind und für Wasserstoff, Fluor, Chlor, Brom, Nitro, Cyano, $C_1$-$C_4$-Alkyl [welches gegebenenfalls durch Fluor und/oder Chlor substituiert ist], $C_1$-$C_4$-Alkoxy [welches gegebenenfalls durch Fluor und/ oder Chlor substituiert ist], für $C_1$-$C_4$-Alkylthio, $C_1$-$C_4$-Alkylsulfinyl oder $C_1$-$C_4$-Alkylsulfonyl [welche gegebenenfalls durch Fluor und/oder Chlor substituiert sind], sowie für Di-($C_1$-$C_4$-alkyl)-aminosulfonyl oder $C_1$-$C_4$-Alkoxy-carbonyl stehen; worin weiter
$R^1$ für den Rest

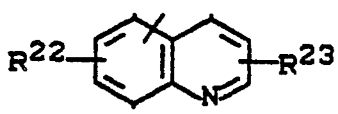

steht, worin
$R^{22}$ und $R^{23}$ gleich oder verschieden sind und für Wasserstoff, Fluor, Chlor, Brom, $C_1$-$C_4$-Alkyl [welches gegebenenfalls durch Fluor und/oder Brom substituiert ist], $C_1$-$C_4$-Alkoxy [welches gegebenenfalls durch Fluor und/oder Chlor substituiert ist], für $C_1$-$C_4$-Alkylthio, $C_1$-$C_4$-Alkylsulfinyl oder $C_1$-$C_4$-Alkylsulfonyl [welche gegebe nenfalls durch Fluor und/oder Chlor substituiert sind], oder für Di-($C_1$-$C_4$-alkyl)-aminosulfonyl stehen;
worin weiter
$R^1$ für den Rest

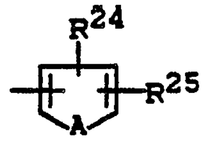

steht, worin

$R^{24}$ und $R^{25}$ gleich oder verschieden sind und für Wasserstoff, Fluor, Chlor, Brom, Cyano, Nitro, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], $C_1$-$C_4$-Alkylthio, $C_1$-$C_4$-Alkylsulfinyl oder $C_1$-$C_4$-Alkylsulfonyl [welches gegebenenfalls durch Fluor und/oder Chlor substituiert ist], Di-($C_1$-$C_4$-alkyl)-aminosulfonyl oder $C_1$-$C_4$-Alkoxy-carbonyl stehen, und

A für Sauerstoff, Schwefel oder die Gruppierung N-$Z^1$ steht, wobei

$Z^1$ für Wasserstoff, $C_1$-$C_4$-Alkyl [welches gegebenenfalls durch Fluor, Chlor, Brom oder Cyano substituiert ist], $C_3$-$C_6$-Cycloalkyl, Benzyl, Phenyl [welches gegebenenfalls durch Fluor, Chlor, Brom oder Nitro substituiert ist], $C_1$-$C_4$-Alkylcar bonyl, $C_1$-$C_4$-Alkoxycarbonyl oder Di-($C_1$-$C_4$-alkyl)-aminocarbonyl steht;

worin weiter

$R^1$ für den Rest

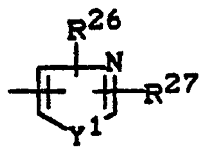

steht, worin

$R^{26}$ für Wasserstoff, $C_1$-$C_5$-Alkyl oder Halogen steht,

$R^{27}$ für Wasserstoff oder $C_1$-$C_5$-Alkyl steht und

$Y^1$ für Schwefel oder die Gruppierung N-$R^{28}$ steht, wobei

$R^{28}$ für Wasserstoff oder $C_1$-$C_5$-Alkyl steht,

worin weiter

$R^1$ für den Rest

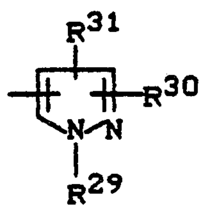

steht, worin

$R^{29}$ für Wasserstoff, $C_1$-$C_4$-Alkyl oder Phenyl steht,

$R^{30}$ für Wasserstoff, Halogen, Cyano, Nitro, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist] oder $C_1$-$C_4$-Alkoxy-carbonyl steht und

$R^{31}$ für Wasserstoff, Halogen oder $C_1$-$C_4$-Alkyl steht,

worin weiter

$R^1$ für den Rest

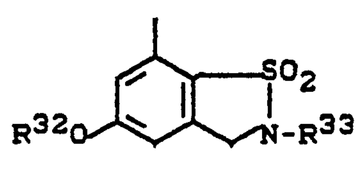

steht, worin

$R^{32}$ für $C_1$-$C_3$-Alkyl steht und
$R^{33}$ für $C_1$-$C_4$-Alkyl steht,
worin weiter
$R^1$ für den Rest

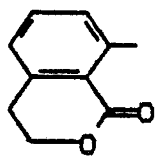

steht,
in welcher weiter
$R^2$ die oben für $R^1$ vorzugsweise angegebene Bedeutung hat, jedoch nicht in jedem Einzelfall mit $R^1$ identisch sein muß, und weiter für Di-($C_1$-$C_3$-alkyl)-amino oder für gegebenenfalls durch Halogen substituiertes $C_1$-$C_8$-Alkyl stehen kann,
in welcher weiter
$R^3$ für Wasserstoff, Fluor, Chlor, Brom, $C_1$-$C_4$-Alkyl, $C_1$-$C_4$-Halogenalkyl, $C_1$-$C_2$-Alkoxy-$C_1$-$C_2$-alkyl, $C_1$-$C_4$-Alkoxy, $C_1$-$C_4$-Halogenalkoxy, $C_1$-$C_4$-Alkylthio, $C_1$-$C_4$-Halogenalkylthio, Amino, $C_1$-$C_4$-Alkylamino, Dimethylamino oder Diethylamino steht,
X für Stickstoff oder eine -CH-Gruppierung steht,
Y für Stickstoff oder eine -$CR^4$-Gruppierung steht, worin
$R^4$ für Wasserstoff, Fluor, Chlor, Brom, Cyano, Methyl, Formyl, Acetyl, Methoxycarbonyl oder Ethoxycarbonyl steht, und
Z für Stickstoff oder eine -$CR^5$-Gruppierung steht, worin
$R^5$ für Wasserstoff, Fluor, Chlor, Brom, $C_1$-$C_4$-Alkyl, $C_1$-$C_4$-Alkoxy, $C_1$-$C_4$-Halogenalkoxy, $C_1$-$C_4$-Alkylthio, $C_1$-$C_4$-Alkylamino, Dimethylamino oder Diethylamino steht, und
M für Wasserstoff oder ein Natrium-, Kalium- oder Calciumäquivalent, ein Ammonium-, ein $C_1$-$C_6$-Alkylammonium-, ein Di-($C_1$-$C_4$-alkyl)-ammonium oder ein Tri-($C_1$-$C_4$-alkyl)-ammonium-äquivalent steht,
wobei N′-(4,6-Dimethyl-pyrimidin-2-yl)-N″-(4-methyl-phenylsulfonylamino)-N‴-(2-chlor-phenylsulfonyl)-guanidin ausgenommen ist.

Gegenstand der Erfindung sind insbesondere Verbindungen der Formel (I), in welcher
$R^1$ für den Rest

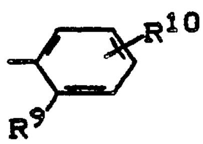

steht, worin
$R^9$ für Fluor, Chlor, Brom, Methyl, Trifluormethyl, Methoxy, Difluormethoxy, Trifluormethoxy, $C_1$-$C_3$-Alkylthio, $C_1$-$C_3$-Alkylsulfinyl, $C_1$-$C_3$-Alkylsulfonyl, Dimethylaminosulfonyl, Diethylaminosulfonyl, N-Methoxy-N-methylaminosulfonyl, Phenyl, Phenoxy oder $C_1$-$C_3$-Alkoxycarbonyl steht und
$R^{10}$ für Wasserstoff steht; worin weiter
$R^1$ für den Rest

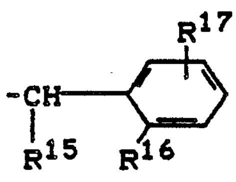

steht, worin
$R^{15}$ für Wasserstoff steht
$R^{16}$ für Fluor, Chlor, Brom, Methyl, Methoxy, Ethoxy, Difluormethoxy, Trifluormethoxy, Methoxycarbonyl,

Ethoxycarbonyl, Methylsulfonyl oder Dimethylaminosulfonyl steht und
$R^{17}$ für Wasserstoff steht; worin weiter
$R^1$ für den Rest

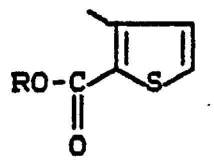

steht, worin
R für $C_1$-$C_2$-Alkyl steht, oder
$R^1$ für den Rest

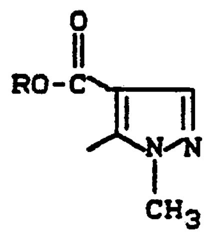

steht, worin
R für $C_1$-$C_2$-Alkyl steht;
in welcher weiter
$R^2$ für gegebenenfalls durch Fluor oder Chlor substituiertes $C_1$-$C_4$-Alkyl oder für gegebenenfalls durch Fluor, Chlor, Brom, $C_1$-$C_3$-Alkyl, Trifluormethyl, Chlordifluormethyl, Methoxy, Ethoxy, Difluormethoxy, Trifluormethoxy, Amino, Acetylamino, Methoxycarbonyl und/oder Ethoxycarbonyl substituiertes Phenyl steht,
$R^3$ für Wasserstoff, Fluor, Chlor, Brom, Methyl, Trifluormethyl, Methoxy, Ethoxy, Difluormethoxy, Methylthio, Ethylthio, Amino, Methylamino, Ethylamino, Dimethylamino oder Diethylamino steht,
X für Stickstoff oder eine -CH-Gruppierung steht,
Y für Stickstoff oder eine -$CR^4$-Gruppierung steht, worin
$R^4$ für Wasserstoff, Fluor, Chlor oder Methyl steht,
Z für Stickstoff oder eine -$CR^5$-Gruppierung steht, worin
$R^5$ für Wasserstoff, Fluor, Chlor, Brom, Methyl, Ethyl, Methoxy, Ethoxy, Propoxy, Isopropoxy, Difluormethoxy, Methylthio, Ethylthio, Methylamino, Ethylamino, Dimethylamino oder Diethylamino steht, und
M für Wasserstoff oder ein Natrium-, Kalium- oder Calciumäquivalent ein Ammonium- oder ein $C_1$-$C_4$-Alkylammoniumäquivalent steht, wobei N′-(4,6-Dimethyl-pyrimidin-2-yl)-N″-(4-methyl-phenylsulfonylamino)-N‴-(2-chlor-phenylsulfonyl)-guanidin ausgenommen ist.

Verwendet man beispielsweise N′-(4,6-Dimethoxy-s-triazin-2-yl)-N″-methoxy-N″,N‴-bis-(2-brom-phenylsulfonyl)-guanidin und Trifluormethansulfonsäurehydrazid als Ausgangsstoffe, so kann der Reaktionsablauf beim erfindungsgemäßen Verfahren durch folgendes Formelschema skizziert werden:

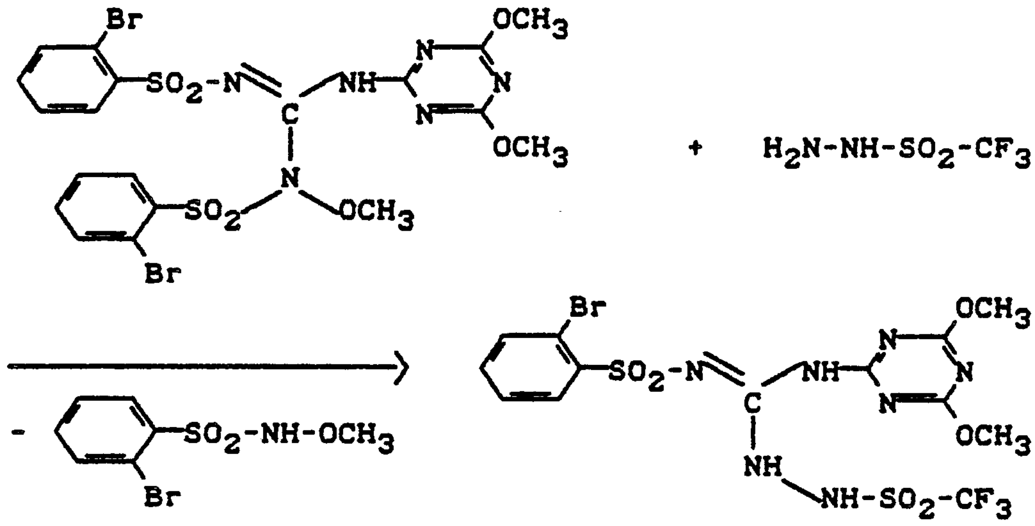

Die beim erfindungsgemäßen Verfahren als Ausgangsstoffe zu verwendenden Sulfonylverbindungen sind durch die Formel (II) allgemein definiert. In Formel (II) haben $R^1$, $R^3$, X, Y und Z vorzugsweise bzw. insbesondere diejenigen Bedeutungen, die bereits oben im Rahmen der Beschreibung der erfindungsgemäßen Verbindungen der Formel (I) als bevorzugt bzw. als insbesondere bevorzugt für $R^1$, $R^3$, X, Y und Z angegeben wurden und

A steht vorzugsweise für eine der nachstehend angegebenen Abgangsgruppen

$R^6$-$SO_2$- N -$OR^7$

oder -Q-$R^8$ , worin

$R^6$ die oben für $R^1$ vorzugsweise angegebene Bedeutung hat, aber nicht in jedem Einzelfall mit $R^1$ identisch sein muß,

$R^7$ für $C_1$-$C_4$-Alkyl, $C_3$-$C_4$-Alkenyl oder Benzyl steht,

$R^8$ für $C_1$-$C_4$-Alkyl, Benzyl oder Phenyl steht und

Q für Sauerstoff oder Schwefel steht.

Insbesondere steht A für die Gruppierung

$R^6$-$SO_2$- N -$OR^7$

, worin

$R^6$ die oben für $R^1$ als insbesondere bevorzugt angegebene Bedeutung hat, aber nicht in jedem Einzelfall mit $R^1$ identisch sein muß und

$R^7$ für Methyl steht.

Beispiele für die Ausgangsstoffe der Formel (II) sind in der nachstehenden Tabelle 1 aufgeführt.

**Tabelle 1: Beispiele für die Ausgangsstoffe der Formel (II)**

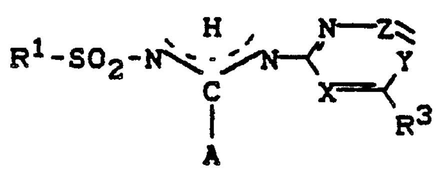

(II)

**Tabelle 1**

| A | $R^1$ | $R^3$ | X | Y | Z |
|---|---|---|---|---|---|
| 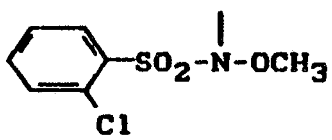 | 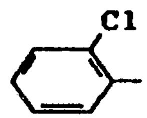 | $CH_3$ | N | CH | $C-OCH_3$ |
| 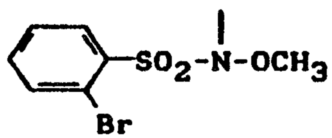 | 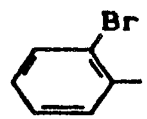 | $OCH_3$ | N | CH | $C-OCH_3$ |
| 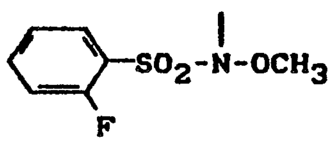 | 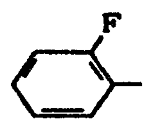 | $OCH_3$ | N | CH | $C-OCH_3$ |
| 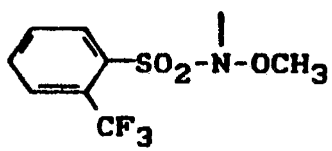 | 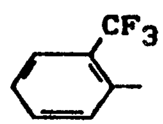 | $OCH_3$ | N | CH | $C-OCH_3$ |
| 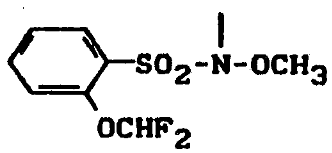 | 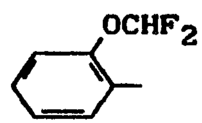 | $OCH_3$ | N | CH | $C-OCH_3$ |

**Tabelle 1** - Fortsetzung

| A | $R^1$ | $R^3$ | X | Y | Z |
|---|---|---|---|---|---|
| 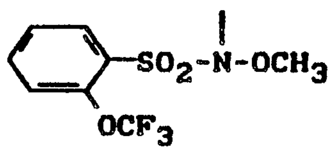 | 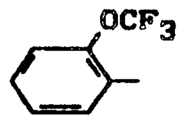 | $OCH_3$ | N | CH | $C-OCH_3$ |
| 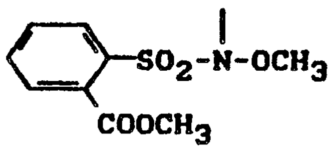 | 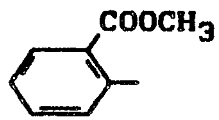 | $CH_3$ | N | CH | $C-CH_3$ |
| 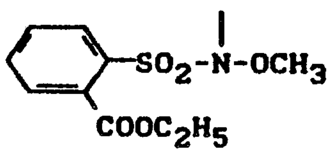 | 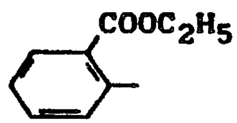 | $CH_3$ | N | CH | $C-CH_3$ |
| 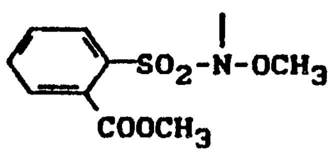 | 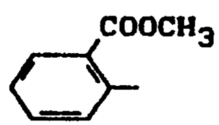 | $OCH_3$ | N | CH | $C-OCH_3$ |
| 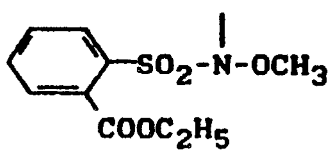 | 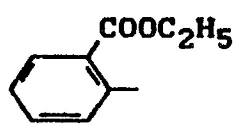 | $OCH_3$ | N | CH | $C-OCH_3$ |

<u>Tabelle 1</u> - Fortsetzung

| A | $R^1$ | $R^3$ | X | Y | Z |
|---|---|---|---|---|---|
| 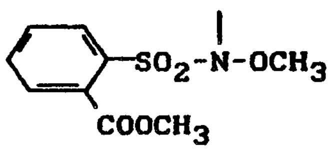 | 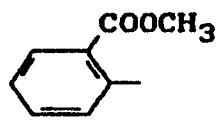 | $C_2H_5$ | N | CH | C-$OCH_3$ |
| 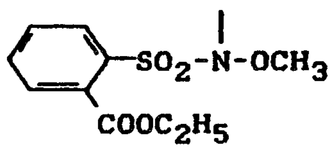 | 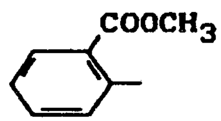 | $C_2H_5$ | N | CH | C-$OCH_3$ |
| 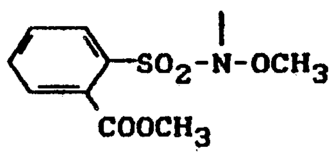 | 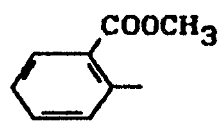 | $OCH_3$ | N | CH | C-Cl |
| 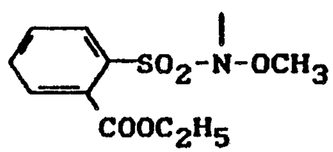 | 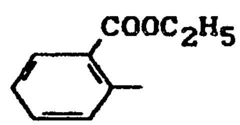 | $OCH_3$ | N | CH | C-Cl |
| 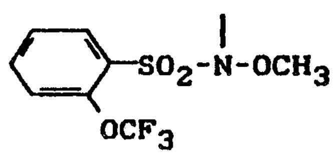 | 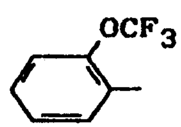 | $OCH_3$ | N | CH | C-Cl |

**Tabelle 1** - Fortsetzung

| A | $R^1$ | $R^3$ | X | Y | Z |
|---|---|---|---|---|---|
| 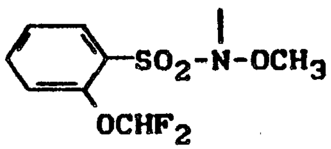 | 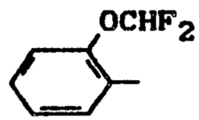 | $OCH_3$ | N | CH | C-Cl |
| 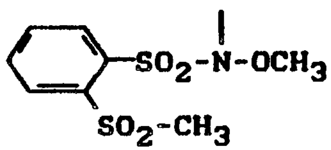 | 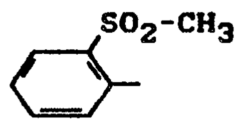 | H | N | CH | $C-CH_3$ |
| 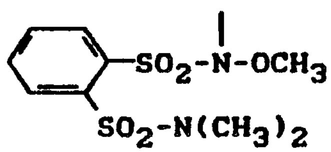 | 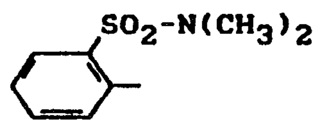 | $CH_3$ | N | CH | $C-OCH_3$ |
| 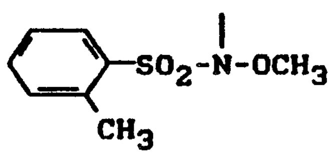 | 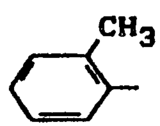 | $OCH_3$ | N | CH | $C-OCH_3$ |
| 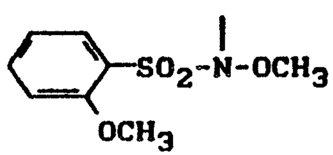 | 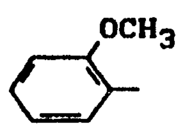 | $CH_3$ | N | CH | $C-OCH_3$ |

Tabelle 1 - Fortsetzung

| A | $R^1$ | $R^3$ | X | Y | Z |
|---|---|---|---|---|---|
| 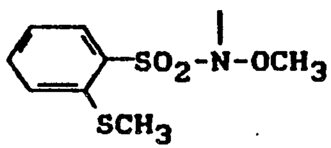 | 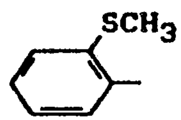 | $OCH_3$ | N | CH | $C-OCH_3$ |
| 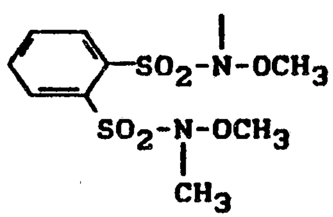 | 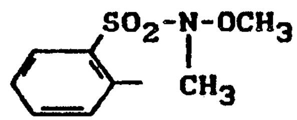 | $OCH_3$ | N | CH | $C-OCH_3$ |
| 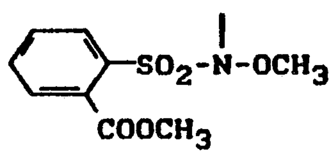 | 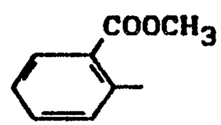 | $CH_3$ | N | CH | $C-OC_2H_5$ |
| 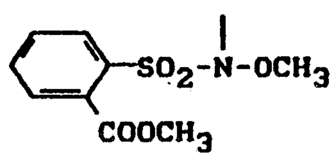 | 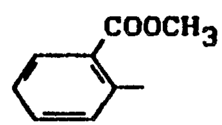 | $OCHF_2$ | N | CH | $C-CH_3$ |

**Tabelle 1** - Fortsetzung

| A | $R^1$ | $R^3$ | X | Y | Z |
|---|---|---|---|---|---|
| 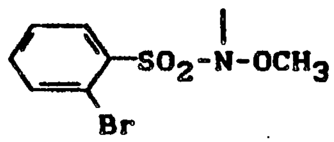 | 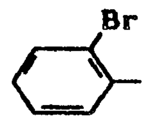 | $CH_3$ | N | CH | $C-SCH_3$ |
| 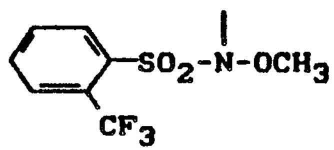 | 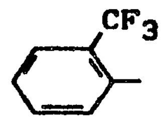 | $CH_3$ | N | CH | $C-N(CH_3)_2$ |
| 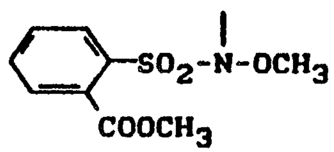 | 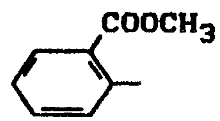 | $OCHF_2$ | N | CH | $C-OCHF_2$ |
| 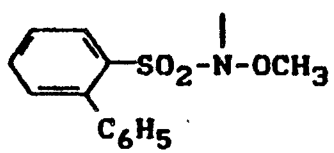 | 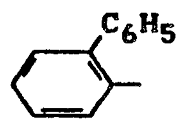 | $OCH_3$ | N | CH | $C-OCH_3$ |
| 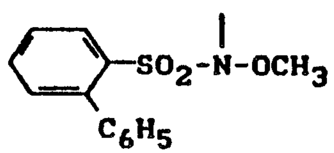 | 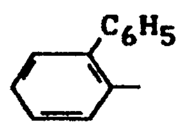 | $OCH_3$ | N | N | $C-OCH_3$ |

**Tabelle 1** - Fortsetzung

| A | $R^1$ | $R^3$ | X | Y | Z |
|---|---|---|---|---|---|
| 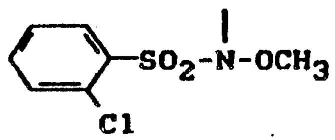 | 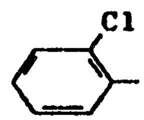 | $CH_3$ | N | N | $C-OCH_3$ |
| 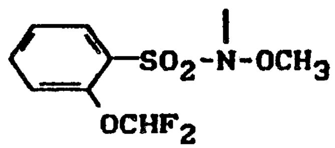 | 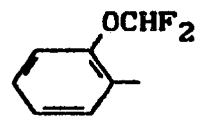 | $OCH_3$ | N | N | $C-OCH_3$ |
| 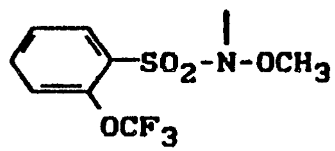 | 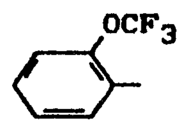 | $OCH_3$ | N | N | $C-OCH_3$ |
| 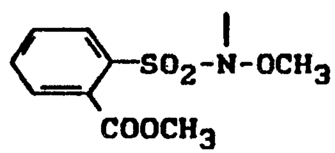 | 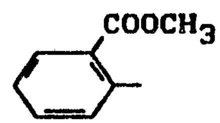 | $CH_3$ | N | N | $C-OCH_3$ |
| 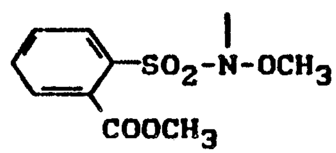 | 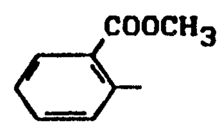 | $OCH_3$ | N | N | $C-OCH_3$ |

**Tabelle 1** - Fortsetzung

| A | $R^1$ | $R^3$ | X | Y | Z |
|---|---|---|---|---|---|
| 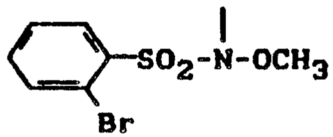 | 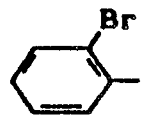 | $CH_3$ | N | N | $C-CH_3$ |
| 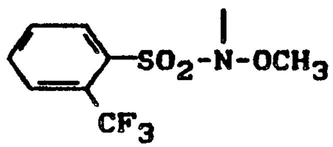 | 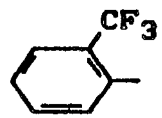 | $CH_3$ | N | N | C-Cl |
| 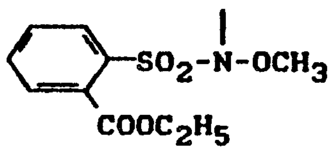 | 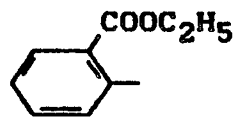 | $OCH_3$ | N | N | $C-OCH_3$ |
| 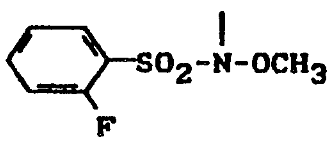 | 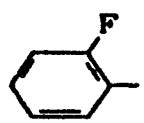 | $OCH_3$ | N | N | $C-OCH_3$ |
| 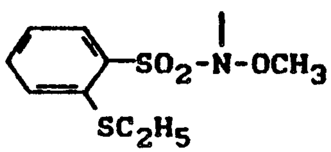 | 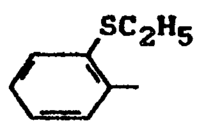 | $OCH_3$ | N | N | $C-OCH_3$ |

**Tabelle 1** - Fortsetzung

| A | $R^1$ | $R^3$ | X | Y | Z |
|---|---|---|---|---|---|
| 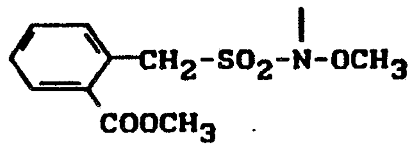 | 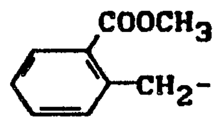 | $OCH_3$ | N | CH | $C-OCH_3$ |
| 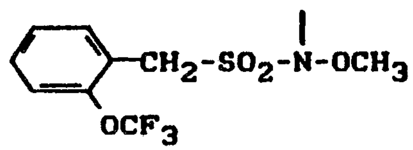 | 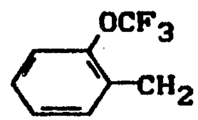 | $OCH_3$ | N | CH | $C-OCH_3$ |
| $-OC_6H_5$ | 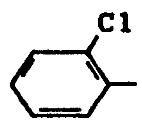 | $CH_3$ | N | CH | $C-CH_3$ |
| $-OCH_3$ | 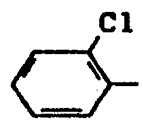 | $CH_3$ | N | CH | $C-OCH_3$ |
| $-SCH_3$ | 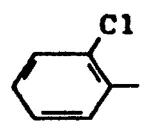 | $OCH_3$ | N | CH | $C-OCH_3$ |

**Tabelle 1** - Fortsetzung

| A | $R^1$ | $R^3$ | X | Y | Z |
|---|---|---|---|---|---|
| $-SC_6H_5$ | 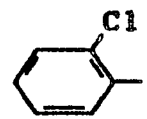 | $OCH_3$ | N | CH | $C-OCH_3$ |
| 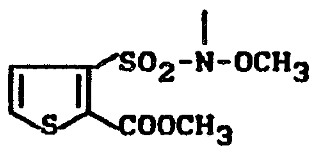 | 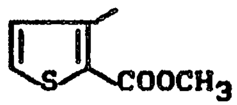 | $CH_3$ | N | N | $C-OCH_3$ |
| 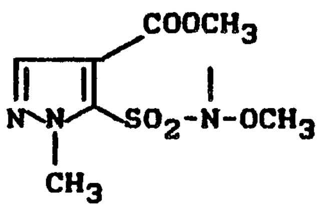 | 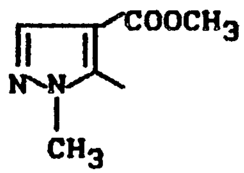 | $OCH_3$ | N | CH | $C-OCH_3$ |
| 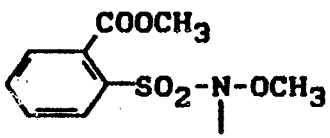 | 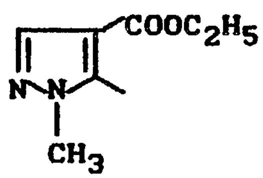 | $OCH_3$ | N | CH | $C-OCH_3$ |

Die Ausgangsstoffe der Formel (II) sind bekannt und/oder können nach an sich bekannten Verfahren hergestellt werden (vgl. EP-A 121 082, EP-A 172 957, EP-A 173 321, EP-A 173 956, EP-A 224 078, EP-A 5 986 und EP-A 24 215).

Die beim erfindungsgemäßen Verfahren weiter als Ausgangsstoffe zu verwendenden Sulfonsäurehydrazide sind durch die Formel (III) allgemein definiert. In Formel (III) hat $R^2$ vorzugsweise bzw. insbesondere diejenige Bedeutung, die bereits oben im Rahmen der Beschreibung der erfindungsgemäßen Verbindungen der Formel (I) als bevorzugt bzw. als insbesondere bevorzugt für $R^2$ angegeben wurde.

Als Beispiele für die Verbindungen der Formel (III) seien genannt:
Methan-, Ethan-, Propan- und Butan-sulfonsäurehydrazid, Trifluormethan-, Perfluorbutan-, Chlormethan- und 2-Chlor-ethan-sulfonsäurehydrazid, Benzolsulfonsäurehydrazid, 2-Fluor-, 4-Fluor-, 2-Chlor-, 4-Chlor-, 2-Brom-, 4-Brom-, 2-Methyl-, 4-Methyl-, 2-Trifluormethyl-, 3-Chlordifluormethyl-, 2-Methoxy-, 4-Methoxy-, 2-Ethoxy-, 4-Ethoxy-, 2-Difluormethoxy-, 2-Trifluormethoxy-, 4-Trifluormethoxy-, 3-Trifluormethoxy-, 2-Methoxycarbonyl-, 4-Methoxycarbonyl-, 2-Ethoxycarbonyl-, 4-Ethoxycarbonylbenzolsulfonsäurehydrazid und Dimethylsulfamoylhydrazin.

Die Ausgangsstoffe der Formel (III) sind bekannt und/oder können nach an sich bekannten Verfahren hergestellt werden (vgl. Org. Synth. 40 (1960), 93 - 95).

Das erfindungsgemäße Verfahren zur Herstellung der neuen Sulfonylaminoguanidinoazine der Formel (I) wird vorzugsweise unter Verwendung von Verdünnungsmitteln durchgeführt. Als Verdünnungsmittel kommen dabei vorzugsweise Wasser und/oder polare organische Lösungsmittel, wie Methanol, Ethanol, Isopropanol, Butanol, Isobutanol, sec-Butano, tert-Butano, Glycoldimethylether, Diglycoldimethylether, Tetrahydrofuran, Dioxan, Essigsäuremethylester, Essigsäureethylester, Acetonitril, Propionitril, Dimethylformamid, Dimethylacetamid, N-Methylpyrrolidon, Dimethylsulfoxid und Tetramethylensulfon, in Betracht.

Die Reaktionstemperaturen können bei dem erfindungsgemäßen Verfahren in einem größeren Bereich variiert werden. Im allgemeinen arbeitet man bei Temperaturen zwischen 0 °C und 150 °C, vorzugsweise bei Temperaturen zwischen 10 °C und 100 °C.

Zur Durchführung des erfindungsgemäßen Verfahrens setzt man je Mol Sulfonylverbindung der Formel (II) im allgemeinen zwischen 1 und 5 Mol, vorzugsweise zwischen 1 und 3 Mol, Sulfonsäurehydrazid der Formel (III) ein.

Im allgemeinen werden die Reaktionskomponenten bei Raumtemperatur oder unter Eiskühlung zusammen gegeben und das Reaktionsgemisch wird, gegebenenfalls bei erhöhter Temperatur, bis zum Ende der Umsetzung gerührt. Die Produkte der Formel (I) fallen im allgemeinen nach dem Abkühlen kristallin an und können durch Absaugen isoliert werden.

Zur Überführung in Salze werden die Verbindungen der Formel (I), in welcher M für Wasserstoff steht, mit geeigneten Metallverbindungen, wie z. B. Natrium- oder Kalium-hydroxid, -methylat oder -ethylat bzw. mit geeigneten Aminen, wie z. B. Isopropylamin, in geeigneten Verdünnungsmitteln, wie z B. Wasser, Methanol oder Ethanol, verrührt. Die Salze können dann - gegebenenfalls nach Einengen - als kristalline Produkte isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die neuen Wirkstoffe eignen sich zur selektiven Bekämpfung von monokotylen und dikotylen Unkräutern im Vorauflauf- und im Nachauflauf-Verfahren, insbesondere in monokotylen Kulturen, besonders auch im Wasserreis.

Bei der Applikation der neuen Wirkstoffe wurde auch Defoliantwirkung und Wirksamkeit gegen Reiskrankheiten, wie Pyricularia oryzae, beobachtet.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.b. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol,

Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N′-dimethylharnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage, ferner auch 2,4-Dichlorphenoxyessigsäure; 4-(2,4-Dichlorphenoxy)-buttersäure; 2,4-Dichlorphenoxypropionsäure; 2-[[[[(4,6-Dimethoxypyrimidin-2-yl)-amino]-carbonyl]-amino]-sulfonyl]-methyl]-benzoesäuremethylester; 3-Isopropyl-2,1,3-benzo thiadiazin-4-on-2,2-dioxid; Methyl-5-(2,4-dichlorphenoxy)-2-nitrobenzoat; 3,5-Dibrom-4-hydroxy-benzonitril; N-(Butoxymethyl)-2-chlor-N-(2,6-diethylphenyl)-acetamid; 2-Chlor-N-{[(4-methoxy-6-methyl-1,3,5-triazin-2-yl)-amino]-carbonyl}-benzolsulfonamid; N,N-Dimethyl-N′-(3-chlor-4-methylphenyl)-harnstoff; 2-[4-(2,4-Dichlorphenoxy)-phenoxy]-propionsäure, deren Methyl- oder deren Ethylester; 3,6-Dichlor-2-pyridincarbonsäure; 4-Amino-6-t-butyl-3-ethylthio-1,2,4-triazin-5(4H)-on; 2-{4-[(6-Chlor-2-benzoxazolyl)-oxy]-phenoxy}-propansäure, deren Methyl- oder deren Ethylester; 2-[4-(3,5-Dichlorpyrid-2-yloxy)-phenoxy]-propionsäure-(trimethylsilylmethylester); 2-(4,5-Dihydro-4-methyl-4-isopropyl-5-oxo-1H-imidazol-2-yl)-pyridin-3-carbonsäure; 3,5-Diiod-4-hydroxybenzonitril; N,N-Dimethyl-N′-(4-isopropylphenyl)-harnstoff; (2-Methyl-4-chlorphenoxy)-essigsäure; (4-Chlor-2-methylphenoxy)-propionsäure; 2-{[[((4-Methoxy-6-methyl-1,3,5-triazin-2-yl)-amino)-carbonyl]-amino]-sulfonyl}-benzoesäure oder deren Methylester; S-Ethyl-N,N-hexamethylen-thiolcarbamat; 4-(Di-n-propylamino)-3,5-dinitrobenzolsulfonamid; N-(1-Ethylpropyl)-3,4-dimethyl-2,6-dinitroanilin; α-Chlor-2′,6′-diethyl-N-(2-propoxyethyl)-acetanilid; 2-Chlor-N-isopropylacetanilid; 4-Ethylamino-2-t-butylamino-6-methylthio-s-triazin; 3-[[[[(4-Methoxy-6-methyl-1,3,5-triazin-2-yl)-amino]-carbonyl]-amino]-sulfonyl]-thiophen-2-carbonsäure-methylester; N,N-Diisopropyl-S-(2,3,3-trichlorallyl)-thiolcarbamat und 3,5,6-Trichlor-2-pyridyloxyessigsäure. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

## Herstellungsbeispiele

Beispiel 1

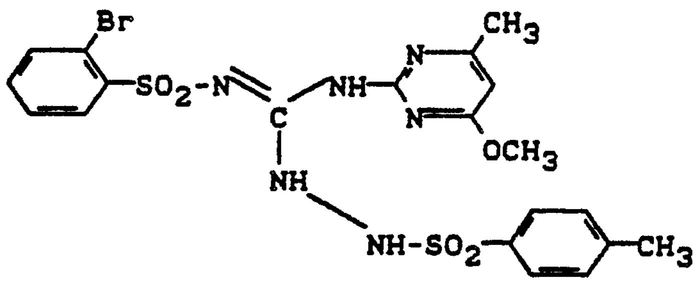

Eine Mischung aus 6,5 g (0,01 Mol) N′-(4-Methoxy-6-methyl-pyrimidin-2-yl)-N″-methoxy-N″,N‴-bis-(2-brom-phenylsulfonyl)-guanidin, 5,6 g (0,03 Mol) p-Toluolsulfonsäurehydrazid, 30 ml Ethanol und 10 ml Wasser wird 60 Minuten unter Rückfluß zum Sieden erhitzt und anschließend 20 Stunden bei 20 °C gerührt. Das kristallin angefallene Produkt wird durch Absaugen isoliert.

Man erhält 3,1 g (51 % der Theorie) N′-(4-Methoxy-6-methyl-pyrimidin-2-yl)-N″-(4-methyl-phenylsulfonylamino)-M‴-(2-brom-phenylsulfonyl)-guanidin vom Schmelzpunkt 156 °C.

Analog Beispiel 1 und entsprechend der allgemeinen Beschreibung des erfindungsgemäßen Verfahrens können die in der nachstehenden Tabelle 2 aufgeführten Verbindungen der Formel (I) hergestellt werden.

**Tabelle 2**: Beispiele für die Verbindungen der Formel (I)

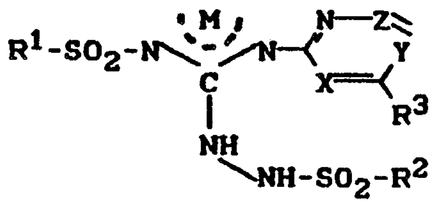

(I)

| Bsp.-Nr. | M | $R^1$ | $R^2$ | $R^3$ | X | Y | Z | Schmelzpunkt (°C) |
|---|---|---|---|---|---|---|---|---|
| 2 | H | 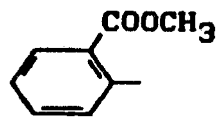 | 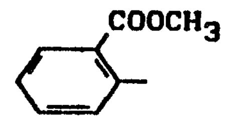 | $CH_3$ | N | CH | $C-CH_3$ | 153 |
| 3 | H | 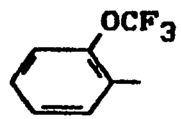 | 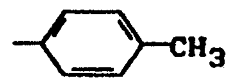 | $OCH_3$ | N | CH | $C-OCH_3$ | 163 |
| 4 | H | 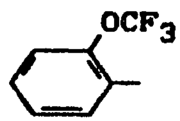 | 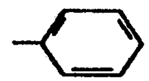 | $OCH_3$ | N | CH | $C-OCH_3$ | 110 |
| 5 | H | 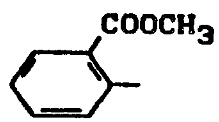 | 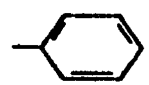 | $OCH_3$ | N | CH | $C-OCH_3$ | 174 |
| 6 | H | 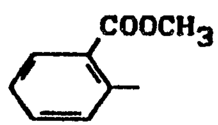 | 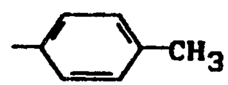 | $OCH_3$ | N | CH | $C-OCH_3$ | 177 |

**Tabelle 2** - Fortsetzung

| Bsp.-Nr. | M | $R^1$ | $R^2$ | $R^3$ | X | Y | Z | Schmelzpunkt (°C) |
|---|---|---|---|---|---|---|---|---|
| 7 | H | 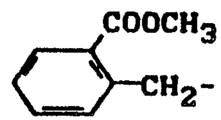 | 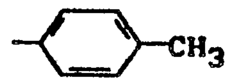 | $OCH_3$ | N | CH | $C-OCH_3$ | 166 |
| 8 | H | 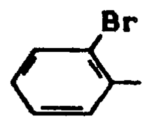 | 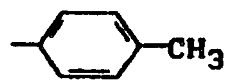 | $OCH_3$ | N | CH | $C-OCH_3$ | 102 |
| 9 | H | 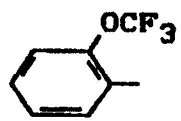 | $CH_3$ | $OCH_3$ | N | CH | $C-OCH_3$ | 174 |
| 10 | H | 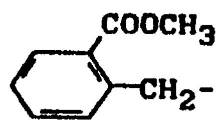 | $CH_3$ | $OCH_3$ | N | CH | $C-OCH_3$ | 87 |
| 11 | H | 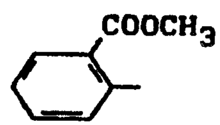 | $CH_3$ | $OCH_3$ | N | CH | $C-OCH_3$ | 184 |

<u>Tabelle 2</u> - Fortsetzung

| Bsp.-Nr. | M | $R^1$ | $R^2$ | $R^3$ | X | Y | Z | Schmelzpunkt (°C) |
|---|---|---|---|---|---|---|---|---|
| 12 | H | 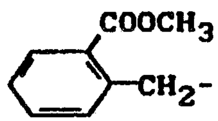 | 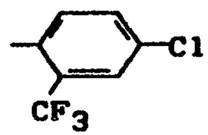 | $OCH_3$ | N | CH | $C-OCH_3$ | 113 |
| 13 | H | 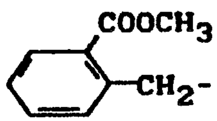 | $C_3H_7$ | $OCH_3$ | N | CH | $C-OCH_3$ | 155 |
| 14 | H | 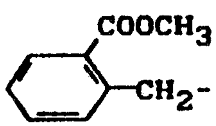 | 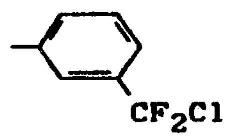 | $OCH_3$ | N | CH | $C-OCH_3$ | 147 |
| 15 | H | 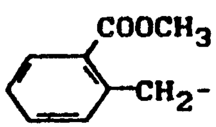 | 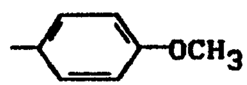 | $OCH_3$ | N | CH | $C-OCH_3$ | 151 |
| 16 | Na | 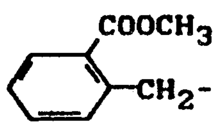 | 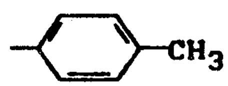 | $OCH_3$ | N | CH | $C-OCH_3$ | |

**Tabelle 2 - Fortsetzung**

| Bsp.-Nr. | M | $R^1$ | $R^2$ | $R^3$ | X | Y | Z | Schmelzpunkt (°C) |
|---|---|---|---|---|---|---|---|---|
| 17 | K | 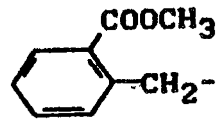 | 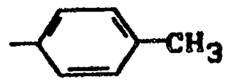 | $OCH_3$ | N | CH | $C-OCH_3$ | |
| 18 | H | 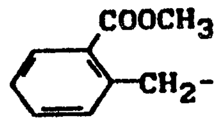 | 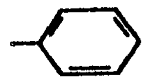 | $OCH_3$ | N | CH | $C-OCH_3$ | |
| 19 | H | 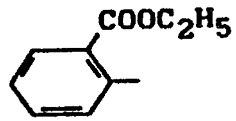 | 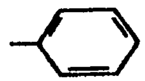 | $OCH_3$ | N | CH | $C-OCH_3$ | |
| 20 | H | 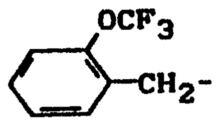 | 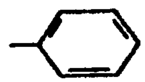 | $OCH_3$ | N | CH | $C-OCH_3$ | |
| 21 | H | 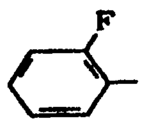 | 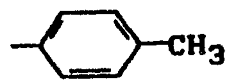 | $CH_3$ | N | CH | $C-OCH_3$ | 154 |
| 22 | H | 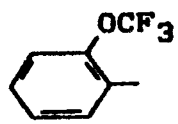 | 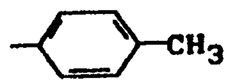 | $CH_3$ | N | CH | $C-OCH_3$ | 179 |

Tabelle 2 - Fortsetzung

| Bsp.-Nr. | M | $R^1$ | $R^2$ | $R^3$ | X | Y | Z | Schmelzpunkt (°C) |
|---|---|---|---|---|---|---|---|---|
| 23 | $H_3NCH(CH_3)_2$ | 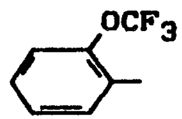 | 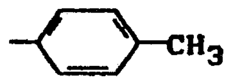 | $CH_3$ | N | CH | $C-OCH_3$ | 84 |
| 24 | K | 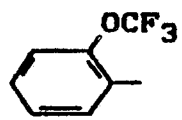 | 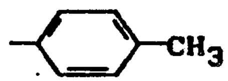 | $CH_3$ | N | CH | $C-OCH_3$ | 127 |
| 25 | H | 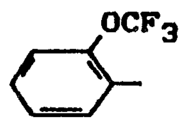 | 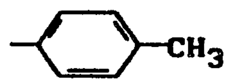 | $CH_3$ | N | CH | $C-OC_2H_5$ | 155 |
| 26 | H | 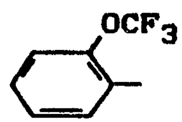 | $CH_3$ | $CH_3$ | N | CH | $C-OCH_3$ | 178 |
| 27 | H | 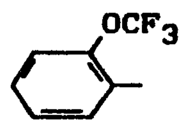 | 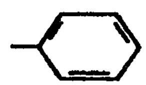 | $CH_3$ | N | CH | $C-OCH_3$ | 182 |
| 28 | H | 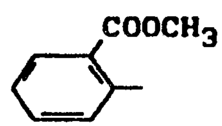 | 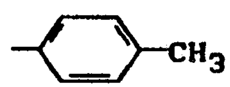 | $CH_3$ | N | CH | $C-OC_2H_5$ | |

**Tabelle 2 - Fortsetzung**

| Bsp.-Nr. | M | $R^1$ | $R^2$ | $R^3$ | X | Y | Z | Schmelzpunkt (°C) |
|---|---|---|---|---|---|---|---|---|
| 29 | H | 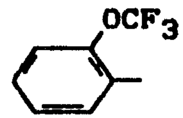 | 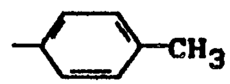 | $C_2H_5$ | N | CH | $C-OCH_3$ | 173 |
| 30 | H | 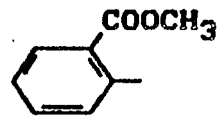 | 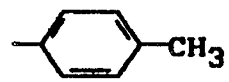 | $CH_3$ | N | CH | $C-OCH_3$ | |
| 31 | H | 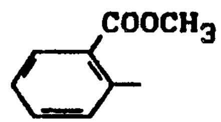 | 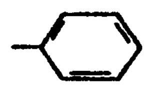 | $CH_3$ | N | CH | $C-OCH_3$ | |
| 32 | H | 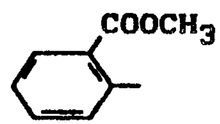 | $CH_3$ | $CH_3$ | N | CH | $C-OCH_3$ | |
| 33 | H | 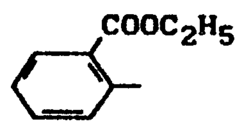 | $CH_3$ | $CH_3$ | N | CH | $C-OCH_3$ | |
| 34 | H | 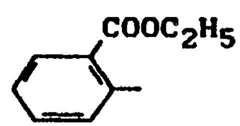 | 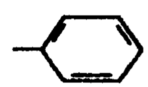 | $CH_3$ | N | CH | $C-OCH_3$ | |

Tabelle 2 - Fortsetzung

| Bsp.-Nr. | M | $R^1$ | $R^2$ | $R^3$ | X | Y | Z | Schmelzpunkt (°C) |
|---|---|---|---|---|---|---|---|---|
| 35 | H | 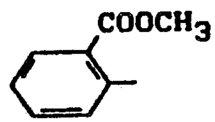 | 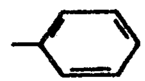 | $CH_3$ | N | N | $C-OCH_3$ | 183 |
| 36 | H | 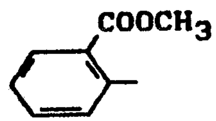 | $CH_3$ | $CH_3$ | N | N | $C-OCH_3$ | |
| 37 | H | 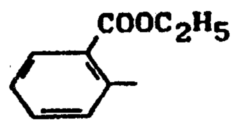 | 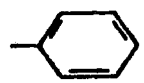 | $OCH_3$ | N | N | $C-OCH_3$ | |
| 38 | H | 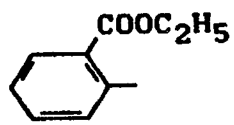 | $CH_3$ | $OCH_3$ | N | N | $C-OCH_3$ | |
| 39 | H | 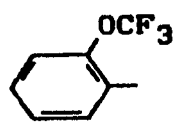 | $CH_3$ | $OCH_3$ | N | N | $C-OCH_3$ | |
| 40 | H | 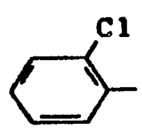 | 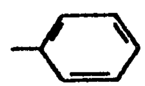 | $OCH_3$ | N | N | $C-OCH_3$ | |

**Tabelle 2** - Fortsetzung

| Bsp.-Nr. | M | $R^1$ | $R^2$ | $R^3$ | X | Y | Z | Schmelzpunkt (°C) |
|---|---|---|---|---|---|---|---|---|
| 41 | H | 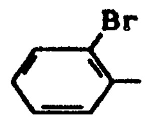 | $CH_3$ | $OCH_3$ | N | N | $C-OCH_3$ | |
| 42 | H | 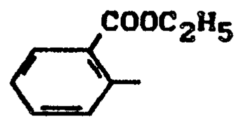 | 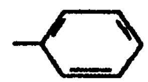 | $CH_3$ | N | N | $C-CH_3$ | |
| 43 | H | 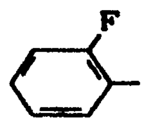 | $CH_3$ | $OCH_3$ | N | N | $C-OCH_3$ | |
| 44 | H | 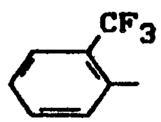 | 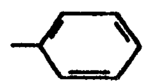 | $OCH_3$ | N | N | $C-OCH_3$ | |
| 45 | H | 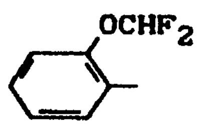 | $CH_3$ | $OCH_3$ | N | N | $C-OCH_3$ | |
| 46 | H | 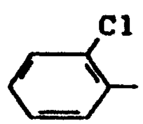 | 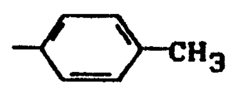 | $CH_3$ | N | N | $C-OCH_3$ | |

**Tabelle 2** - Fortsetzung

| Bsp.-Nr. | M | $R^1$ | $R^2$ | $R^3$ | X | Y | Z | Schmelzpunkt (°C) |
|---|---|---|---|---|---|---|---|---|
| 47 | H | 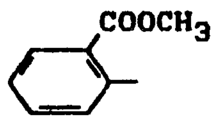 | 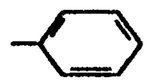 | $CH_3$ | N | N | $C-OCH_3$ | |
| 48 | H | 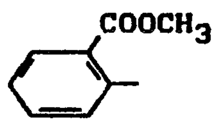 | 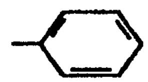 | $OCH_3$ | N | N | $C-OCH_3$ | |
| 49 | H | 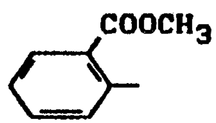 | 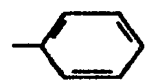 | $C_2H_5$ | N | N | $C-OCH_3$ | |
| 50 | H | 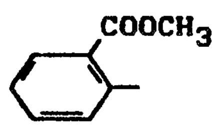 | 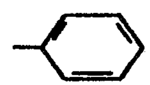 | $CH_3$ | N | N | $C-OC_2H_5$ | |
| 51 | H | 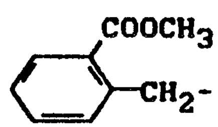 | 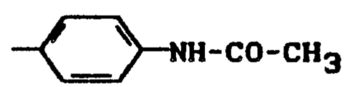 | $OCH_3$ | N | CH | $C-OCH_3$ | 223 |

**Tabelle 2** - Fortsetzung

| Bsp.-Nr. | M | $R^1$ | $R^2$ | $R^3$ | X | Y | Z | Schmelzpunkt (°C) |
|---|---|---|---|---|---|---|---|---|
| 52 | H | 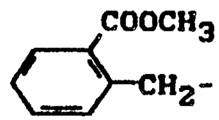 | 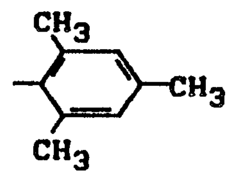 | $OCH_3$ | N | CH | $C-OCH_3$ | 100 |
| 53 | H | 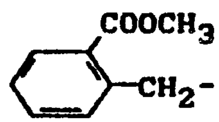 | 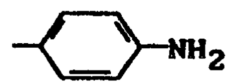 | $OCH_3$ | N | CH | $C-OCH_3$ | 218 |
| 54 | H | 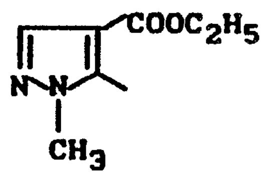 | 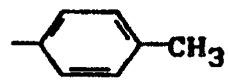 | $OCH_3$ | N | CH | $C-OCH_3$ | 112 |
| 55 | H | 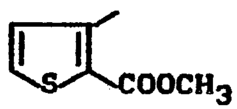 | 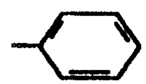 | $CH_3$ | N | N | $C-OCH_3$ | |
| 56 | H | 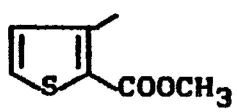 | 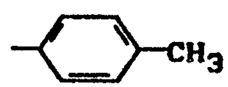 | $OCH_3$ | N | N | $C-OCH_3$ | |

Tabelle 2 - Fortsetzung

| Bsp.-Nr. | M | $R^1$ | $R^2$ | $R^3$ | X | Y | Z | Schmelzpunkt (°C) |
|---|---|---|---|---|---|---|---|---|
| 57 | H | 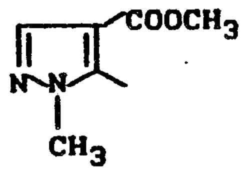 | 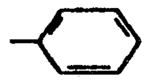 | $OCH_3$ | N | N | $C-OCH_3$ | |
| 58 | H | 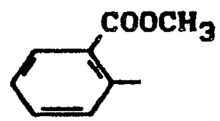 | 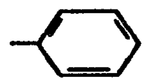 | $CH_3$ | N | CH | $C-CH_3$ | |
| 59 | H | 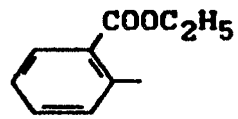 | 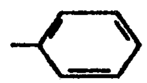 | $CH_3$ | N | CH | $C-CH_3$ | |
| 60 | H | 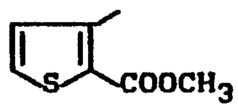 | 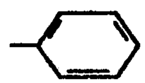 | $OCH_3$ | N | CH | $C-OCH_3$ | |
| 61 | H | 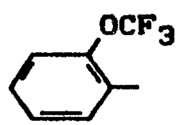 | 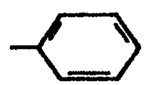 | $CH_3$ | N | CH | $C-CH_3$ | |
| 62 | H | 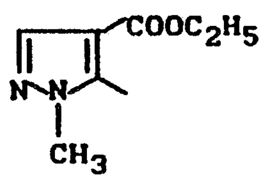 | 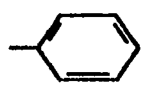 | $OCH_3$ | N | CH | $C-OCH_3$ | |

**Tabelle 2** - Fortsetzung

| Bsp.-Nr. | M | $R^1$ | $R^2$ | $R^3$ | X | Y | Z | Schmelzpunkt (°C) |
|---|---|---|---|---|---|---|---|---|
| 63 | H | 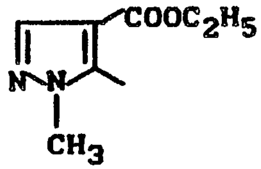 | 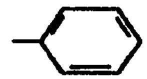 | $OCH_3$ | N | N | $C-OCH_3$ | |
| 64 | H | 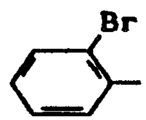 | 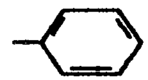 | $OCH_3$ | N | N | $C-OCH_3$ | |
| 65 | H | 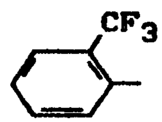 | 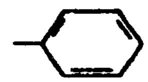 | $CH_3$ | N | CH | $C-CH_3$ | |
| 66 | H | 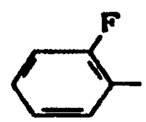 | 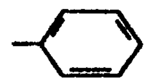 | $CH_3$ | N | CH | $C-CH_3$ | |
| 67 | H | 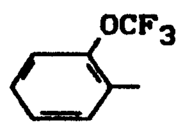 | 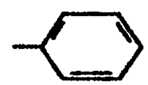 | $CH_3$ | N | CH | $C-CH_3$ | |

Tabelle 2 - Fortsetzung

| Bsp.-Nr. | M | $R^1$ | $R^2$ | $R^3$ | X | Y | Z | Schmelzpunkt (°C) |
|---|---|---|---|---|---|---|---|---|
| 68 | H | 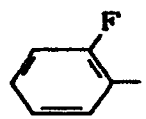 | 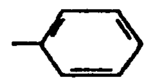 | $OCH_3$ | N | CH | $C-OCH_3$ | |
| 69 | H | 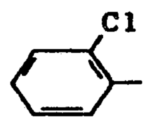 | 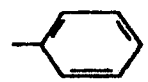 | $OCH_3$ | N | CH | $C-OCH_3$ | |
| 70 | H | 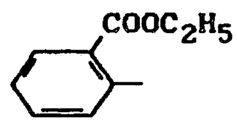 | 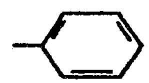 | $OCH_3$ | N | CH | C-Cl | |
| 71 | H | 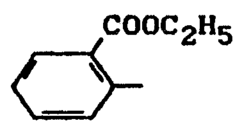 | $-C_3H_7-n$ | $OCH_3$ | N | CH | C-Cl | |
| 72 | H | 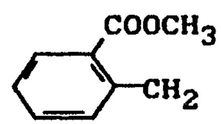 | $N(CH_3)_2$ | $OCH_3$ | N | CH | $C-OCH_3$ | 158 |
| 73 | H | 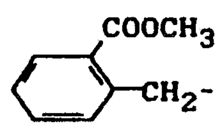 | 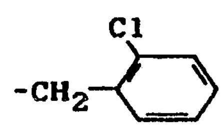 | $OCH_3$ | N | CH | $C-OCH_3$ | |

**Tabelle 2** - Fortsetzung

| Bsp.-Nr. | M | $R^1$ | $R^2$ | $R^3$ | X | Y | Z | Schmelzpunkt (°C) |
|---|---|---|---|---|---|---|---|---|
| 74 | H | 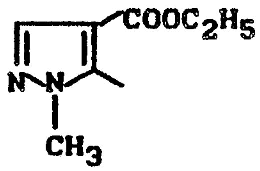 | $CH_3$ | $OCH_3$ | N | CH | $C-OCH_3$ | |
| 75 | H | 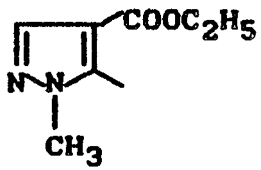 | $-C_3H_7-n$ | $OCH_3$ | N | CH | $C-OCH_3$ | |
| 76 | H | 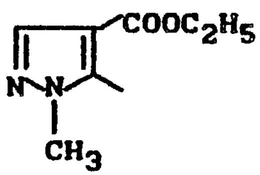 | $-CH_2-$ 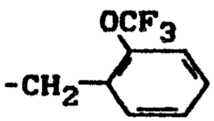 | $OCH_3$ | N | CH | $C-OCH_3$ | |
| 77 | H | 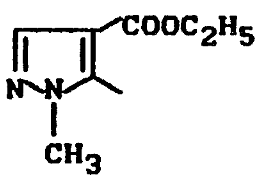 | $N(CH_3)_2$ | $OCH_3$ | N | CH | $C-OCH_3$ | |
| 78 | H | 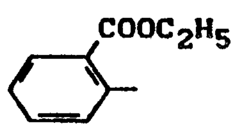 | 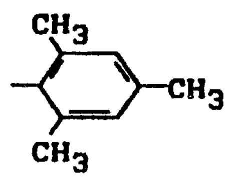 | $OCH_3$ | N | CH | C-Cl | |

**Tabelle 2** - Fortsetzung

| Bsp.-Nr. | M | $R^1$ | $R^2$ | $R^3$ | X | Y | Z | Schmelzpunkt (°C) |
|---|---|---|---|---|---|---|---|---|
| 79 | H | 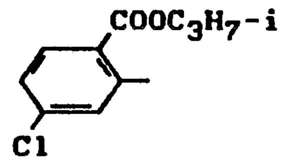 | 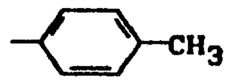 | $CH_3$ | N | N | $C-OCH_3$ | |
| 80 | H | 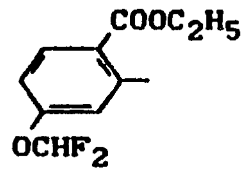 | 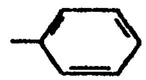 | $OCH_3$ | N | CH | $C-OCH_3$ | |
| 81 | H | 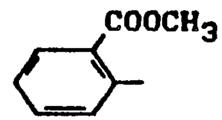 | 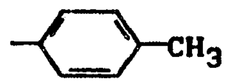 | $OCHF_2$ | N | CH | $C-OCHF_2$ | |
| 82 | H | 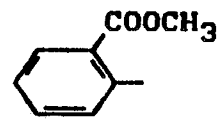 | 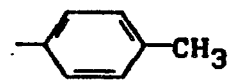 | $OCH_3$ | N | CH | $CH(OCH_3)_2$ | |
| 83 | H | 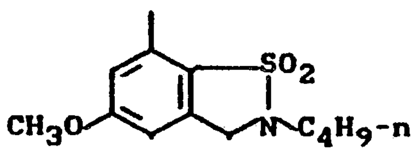 | 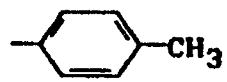 | $OCH_3$ | N | CH | $C-OCH_3$ | |

Tabelle 2 - Fortsetzung

| Bsp.-Nr. | M | $R^1$ | $R^2$ | $R^3$ | X | Y | Z | Schmelzpunkt (°C) |
|---|---|---|---|---|---|---|---|---|
| 84 | H | 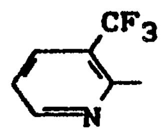 | 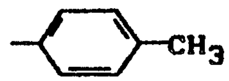 | $OCH_3$ | N | CH | $C-OCH_3$ | |
| 85 | H | 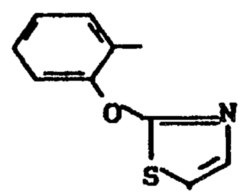 | 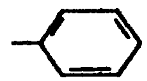 | $OCH_3$ | N | N | $C-OCH_3$ | |
| 86 | H | 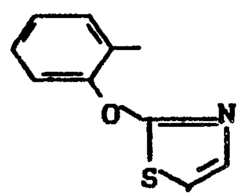 | 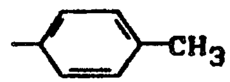 | $OCH_3$ | N | CH | $C-OCH_3$ | |
| 87 | H | 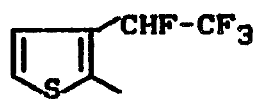 | $-C_3H_7-n$ | $OCH_3$ | N | CH | $C-OCH_3$ | |
| 88 | H | 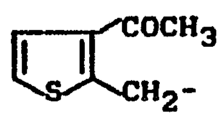 | 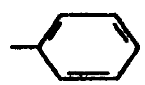 | $OCH_3$ | N | CH | $C-OCH_3$ | |

Tabelle 2 - Fortsetzung

| Bsp.-Nr. | M | $R^1$ | $R^2$ | $R^3$ | X | Y | Z | Schmelz-punkt (°C) |
|---|---|---|---|---|---|---|---|---|
| 89 | H | 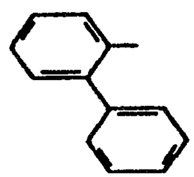 | 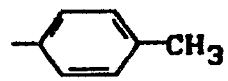 | $OCH_3$ | N | N | $C-OCH_3$ | |
| 90 | H | 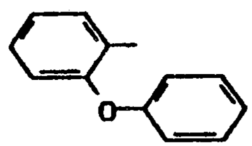 | 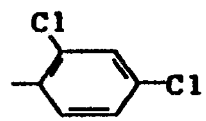 | $OCH_3$ | N | N | $C-OCH_3$ | |
| 91 | H | 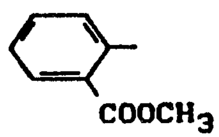 | 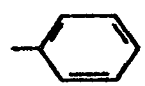 | $NHCH_3$ | N | N | $C-OC_2H_5$ | |
| 92 | H | 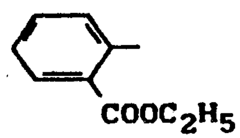 | $CH_3$ | $NHCH_3$ | N | N | $C-OC_2H_5$ | |
| 93 | H | 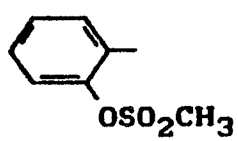 | 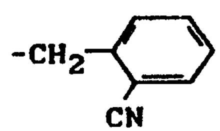 | $NHCH_3$ | N | N | $C-C_2H_5$ | |

Tabelle 2 - Fortsetzung

| Bsp.-Nr. | M | $R^1$ | $R^2$ | $R^3$ | X | Y | Z | Schmelz-punkt (°C) |
|---|---|---|---|---|---|---|---|---|
| 94 | H | 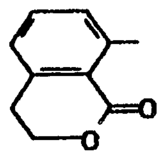 | 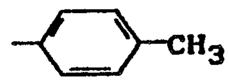 | $NHCH_3$ | N | N | $C-OC_2H_5$ | |
| 95 | H | 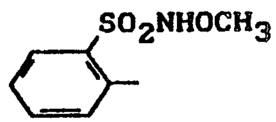 | 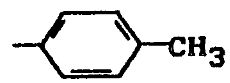 | $OCH_3$ | N | CH | $C-OCH_3$ | 164 |
| 96 | H | 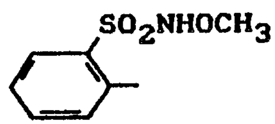 | 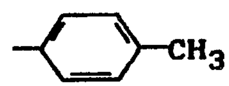 | $CH_3$ | N | CH | $C-OCH_3$ | 202 |
| 97 | H | 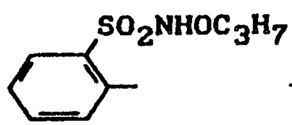 | 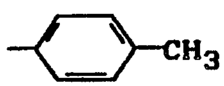 | $CH_3$ | N | CH | $C-OCH_3$ | 181 |
| 98 | H | 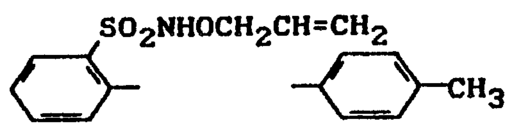 | | $CH_3$ | N | CH | $C-OCH_3$ | 148 |
| 99 | H | 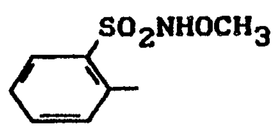 | 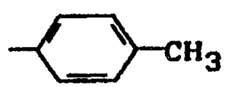 | $CH_3$ | N | CH | $C-OC_2H_5$ | 106 |

**Tabelle 2 - Fortsetzung**

| Bsp.-Nr. | M | $R^1$ | $R^2$ | $R^3$ | X | Y | Z | Schmelzpunkt (°C) |
|---|---|---|---|---|---|---|---|---|
| 100 | H | 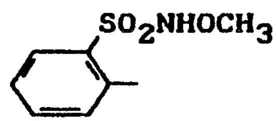 | $CH_3$ | $OCH_3$ | N | CH | $C-OCH_3$ | |
| 101 | H | 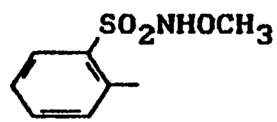 | 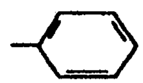 | $OCH_3$ | N | CH | $C-OCH_3$ | |
| 102 | H | 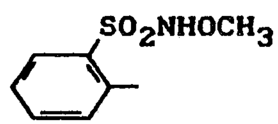 | 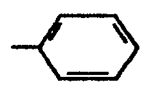 | $OCH_3$ | N | CH | C-Cl | |
| 103 | H | 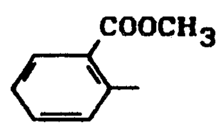 | 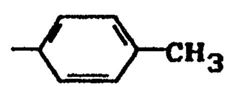 | $CF_3$ | N | CH | $C-OCH_3$ | 63 |

<u>Tabelle 2</u> - Fortsetzung

| Bsp.-Nr. | M | $R^1$ | $R^2$ | $R^3$ | X | Y | Z | Schmelzpunkt (°C) |
|---|---|---|---|---|---|---|---|---|
| 104 | H | 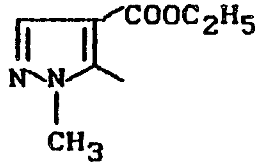 | 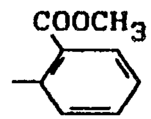 | $CH_3$ | N | CH | $C-CH_3$ | 157 |
| 105 | H | 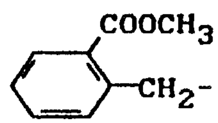 | 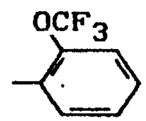 | $OCH_3$ | N | CH | $C-OCH_3$ | 211 |
| 106 | H | 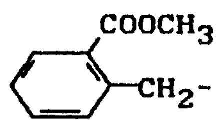 | 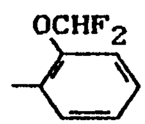 | $OCH_3$ | N | CH | $C-OCH_3$ | 147 |
| 107 | H | 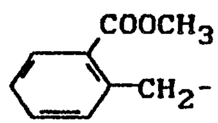 | 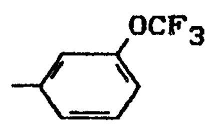 | $OCH_3$ | N | CH | $C-OCH_3$ | |
| 108 | H | 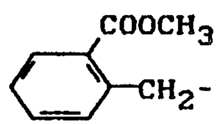 | 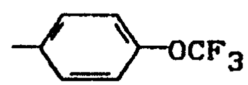 | $OCH_3$ | N | CH | $C-OCH_3$ | 160 |

Tabelle 2 - Fortsetzung

| Bsp.-Nr. | M | $R^1$ | $R^2$ | $R^3$ | X | Y | Z | Schmelzpunkt (°C) |
|---|---|---|---|---|---|---|---|---|
| 109 | H | 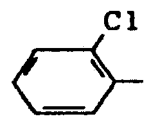 | 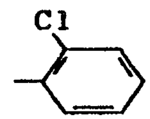 | $CH_3$ | N | CH | $C-OCH_3$ | 168 |
| 110 | H | 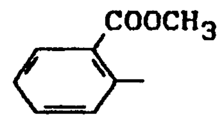 | 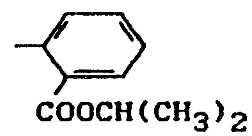 | $CH_3$ | N | CH | $C-CH_3$ | 159 |
| 111 | H | 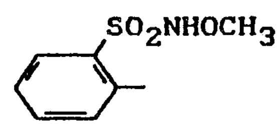 | 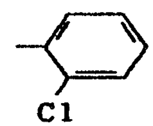 | $OCH_3$ | N | N | $C-OCH_3$ | 188 |
| 112 | H | 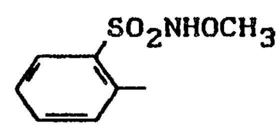 | 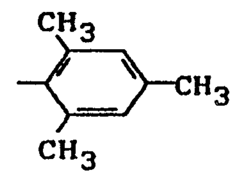 | $OCH_3$ | N | N | $C-OCH_3$ | 176 |
| 113 | H | 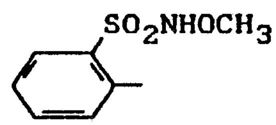 | 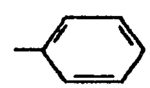 | $OCH_3$ | N | N | $C-OCH_3$ | 198 |

Anwendungsbeispiele

In den folgenden Anwendungsbeispielen wird die nachstehend aufgeführte Verbindung als Vergleichssubstanz herangezogen:

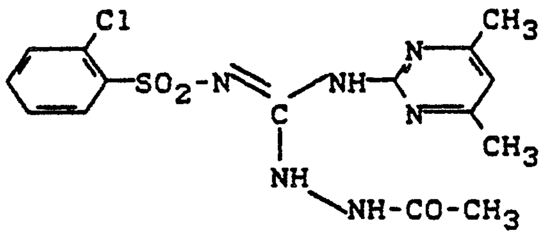

(A)

Die Formeln der für die Anwendungsbeispiele herangezogenen erfindungsgemäßen Verbindungen sind - mit der Numerierung der Herstellungsbeispiele ("Beisp.-Nr.") -nachstehend einzeln aufgeführt.

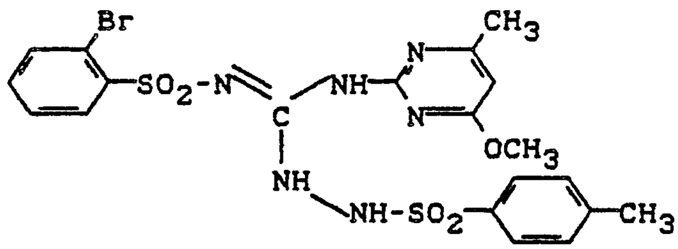

(1)

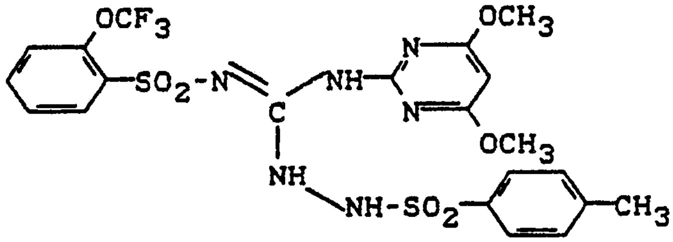

(3)

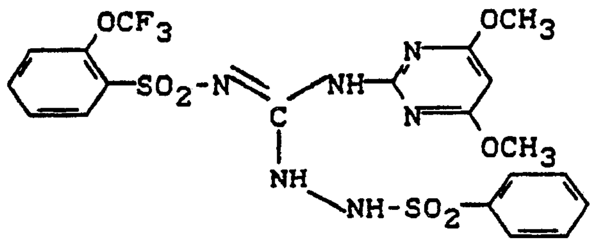

(4)

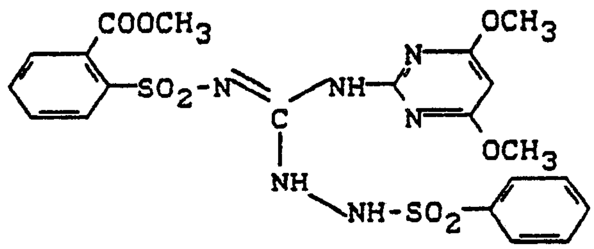

(5)

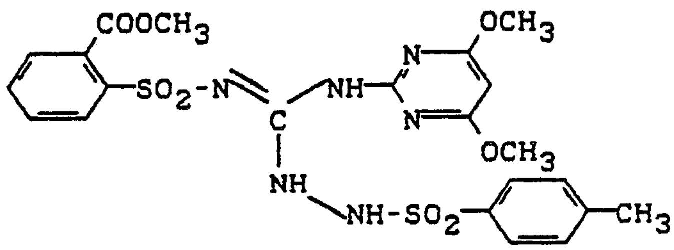

(6)

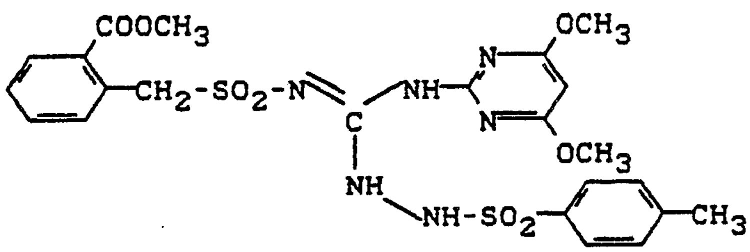

(7)

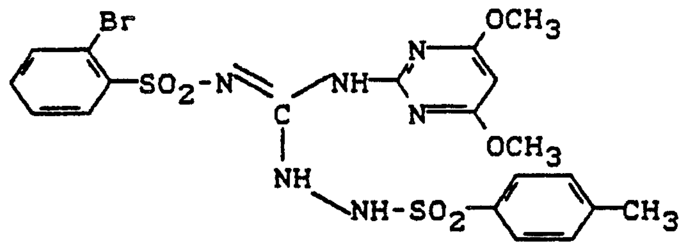

(8)

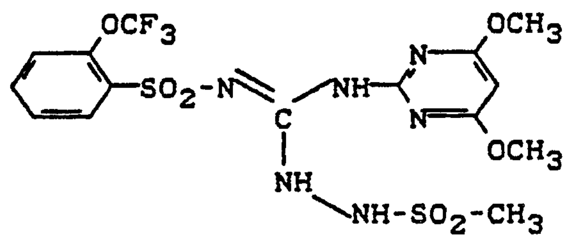

(9)

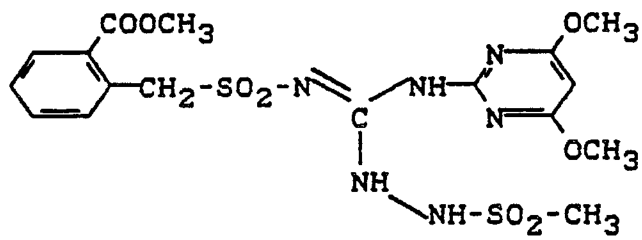

(10)

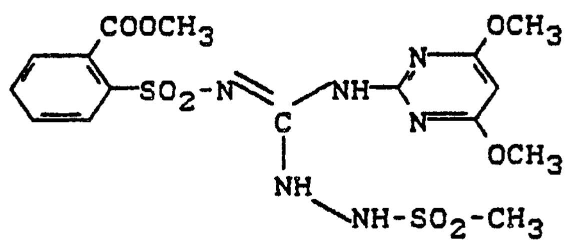

(11)

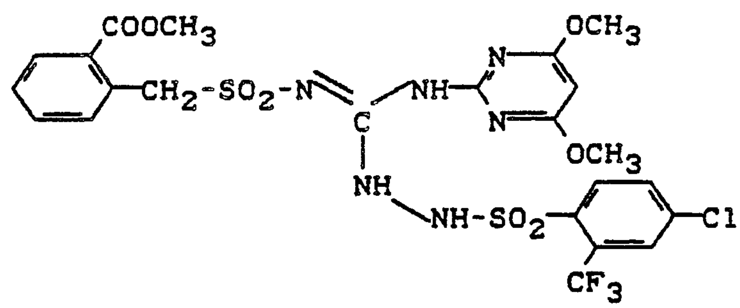

(12)

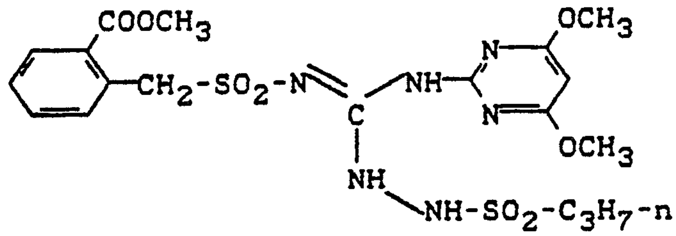

(13)

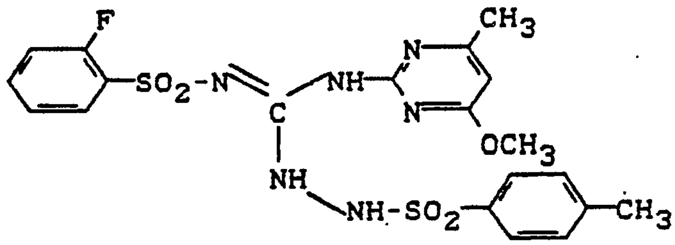

(21)

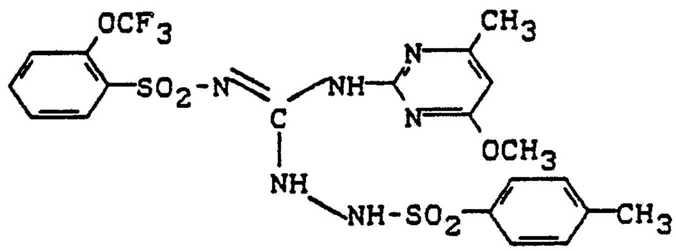

(22)

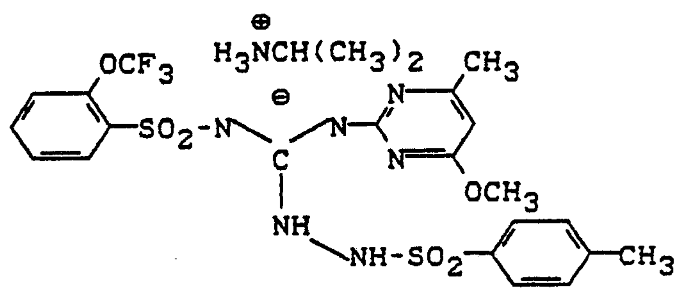

(23)

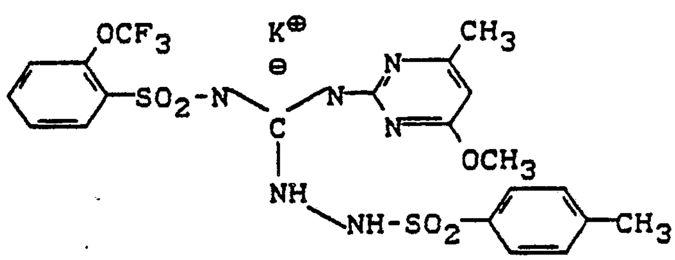

(24)

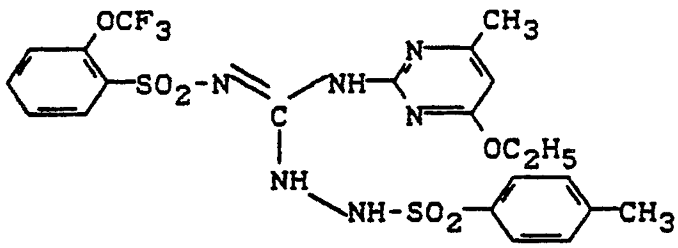

(25)

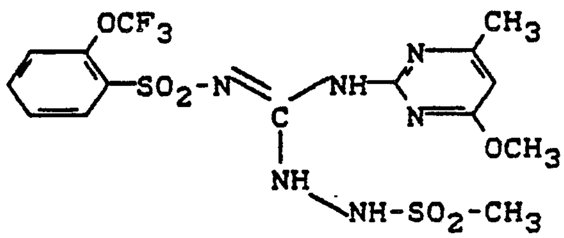

(26)

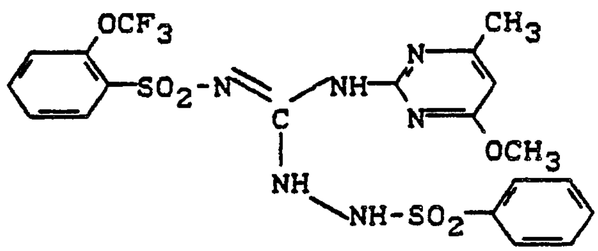

(27)

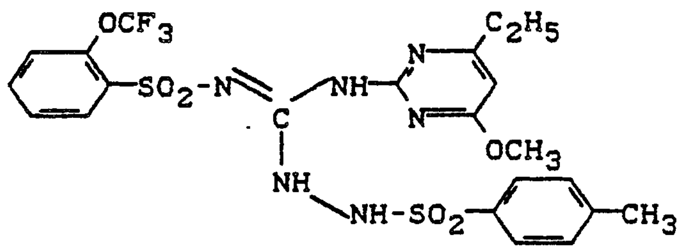

(29)

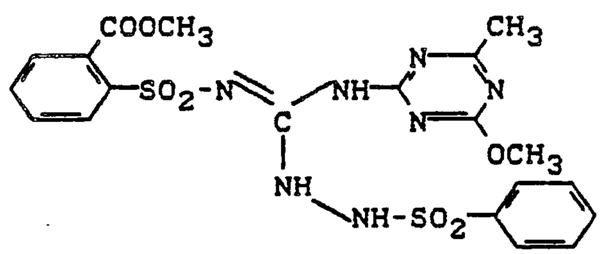

(35)

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)

100 % = totale Vernichtung

In diesem Test zeigen beispielsweise die Verbindungen gemäß den Herstellungsbeispielen (1), (3), (4), (5), (6), (7), (8), (9), (11), (21), (22), (23), (24), (25), (26), (27), (29) und (35) erheblich stärkere Wirkung als die Vergleichssubstanz (A).

Beispiel B

Post-emergence-Test

Lösungsmittel: 5 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)

100 % = totale Vernichtung

In diesem Test zeigen, beispielsweise die Verbindungen gemäß den Herstellungsbeispielen (1), (3), (4), (5), (6), (7), (8), (9), (19), (11), (12), (13), (21), (22), (23), (24), (25), (26), (27), (29) und (35) erheblich stärkere Wirkung als die Vergleichssubstanz (A).

Beispiel C

Test an verpflanztem Wasserreis

Lösungsmittel: 5 Gewichtsteile Aceton

Emulgator: 1 Gewichtsteil Benzyloxypolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat auf die gewünschte Konzentration.

Pflanzgefäße (Oberfläche 1/5000 Ar) werden mit Boden aus einem Reisfeld gefüllt. Zwei Reispflanzen (Sorte: Kinmaze) werden im 2 - 3 Blattstadium (ca. 10 cm hoch) in die Gefäße verpflanzt. Samen von Echinochloa crus galli und/oder Monochoria vaginalis und/oder kleine Rhizomabschnitte von Eleocharis acicularis L. werden in die feucht gehaltene Erde ausgesät. 2 Tage nach dem Verpflanzen des Reises wird der Boden bis zu einer Wassertiefe von 3 cm überstaut. Die Wirkstoffzubereitung wird auf die Wasseroberfläche ausgebracht. Die Wirkstoffkonzentration in der Zubereitung spielt keine Rolle, entscheidend ist nur die Aufwandmenge des Wirkstoffs pro Flächeneinheit.

Nach der Anwendung des Wirkstoffs wird für 2 Tage durch die Pflanzgefäße ein vertikal absteigender Wasserstrom mit einer Geschwindigkeit von 2 - 3 cm pro Tag eingestellt. Danach werden die Testansätze

unter Überflutungsbedingungen gehalten, wobei die Wassertiefe 3 cm beträgt.

Nach 4 Wochen wird der Schädigungsgrad der Pflanzen bonitiert in % Schädigung (bzw. Unkrautwirkung) im Vergleich zu einer unbehandelten Kontrolle

Es bedeuten:

0 % = keine Wirkung (wie unbehandelte Kontrolle)

100 % = totale Vernichtung

In diesem Test zeigt sich die gute Verträglichkeit der erfindungsgemäßen Wirkstoffe - insbesondere der Verbindungen (7) und (10) - in Reis bei gleichzeitig sehr guter Unkrautwirkung, während die sehr ähnliche vorbekannte Verbindung (A) starke Schäden am Reis hervorruft.

## Ansprüche

1. Sulfonylaminoguanidinoazine der allgemeinen Formel (I)

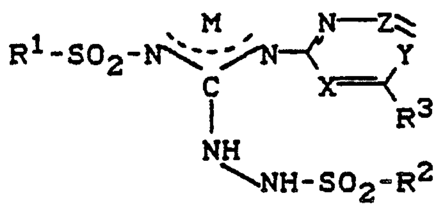

(I)

in welcher

$R^1$ für einen gegebenenfalls substituierten Rest aus der Reihe Alkyl, Aralkyl, Aryl und Hetaryl steht,

$R^2$ für einen gegebenenfalls substituierten Rest aus der Reihe Alkyl, Aralkyl, Aryl, Hetaryl und Dialkylamino steht,

$R^3$ für Wasserstoff, Halogen, Hydroxy, Alkyl, Halogenalkyl, Alkoxyalkyl, Alkoxy, Halogenalkoxy, Alkylthio, Halogenalkylthio, Amino, Alkylamino oder Dialkylamino steht,

X für Stickstoff oder eine -CH-Gruppierung steht,

Y für Stickstoff oder eine $-CR^4$-Gruppierung steht, worin

$R^4$ für Wasserstoff, Halogen, Cyano, Alkyl, Formyl, Alkyl-carbonyl oder Alkoxy-carbonyl steht,

Z für Stickstoff oder eine $-CR^5$-Gruppierung steht, worin

$R^5$ für Wasserstoff, Halogen, Hydroxy, Alkyl, Alkoxy, Halogenalkoxy, Alkylthio, Alkylamino oder Dialkylamino steht, und

M für Wasserstoff, ein Metalläquivalent, ein Ammonium-, ein Alkylammonium-, ein Dialkylammonium- oder ein Trialkylammoniumäquivalent steht,

wobei N′-(4,6-Dimethyl-pyrimidin-2-yl)-N″-(4-methyl-phenylsulfonylamino)-N‴-(2-chlor-phenylsulfonyl)-guanidin ausgenommen ist.

2. Sulfonylaminoguanidinoazine der allgemeinen Formel (I) gemäß Anspruch 1, dadurch gekennzeichnet, daß darin

$R^1$ für den Rest

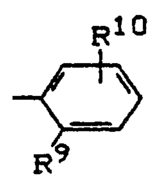

steht, worin

$R^9$ und $R^{10}$ gleich oder verschieden sind und für Wasserstoff, Fluor, Chlor, Brom, Iod, Cyano, Nitro, $C_1$-$C_6$-Alkyl [welches gegebenenfalls durch Fluor, Chlor, Brom, Cyano, Carboxy, $C_1$-$C_4$-Alkoxycarbonyl, $C_1$-$C_4$-Alkylaminocarbonyl, Di-($C_1$-$C_4$-alkyl)-aminocarbonyl, Hydroxy, $C_1$-$C_4$-Alkoxy, Formyloxy, $C_1$-$C_4$-Alkyl-carbonyloxy, $C_1$-$C_4$-Alkoxy-carbonyloxy, $C_1$-$C_4$-Alkylamino-carbonyloxy, $C_1$-$C_4$-Alkylthio, $C_1$-$C_4$-Alkylsulfinyl, $C_1$-$C_4$-Alkylsulfonyl, Di-($C_1$-$C_4$-alkyl)-aminosulfonyl, $C_3$-$C_6$-Cycloalkyl oder Phenyl substituiert ist], für $C_2$-$C_6$-Alkenyl [welches gegebenenfalls durch Fluor, Chlor, Brom, Cyano, $C_1$-$C_4$-Alkoxy-carbonyl, Carboxy oder Phenyl substituiert ist], für $C_2$-$C_6$-Alkinyl [welches gegebenenfalls durch Fluor, Chlor, Brom, Cyano, $C_1$-$C_4$-

Alkoxy-carbonyl, Carboxy oder Phenyl substituiert ist], für $C_1$-$C_4$-Alkoxy [welches gegebenenfalls durch Fluor, Chlor, Brom, Cyano, Carboxy, $C_1$-$C_4$-Alkoxy-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für $C_1$-$C_4$-Alkylthio [welches gegebenenfalls durch Fluor, Chlor, Brom, Cyano, Carboxy $C_1$-$C_4$-Alkoxy-carbonyl, $C_1$-$C_4$-Alkylthio, $C_1$-$C_4$-Alkylsulfinyl oder $C_1$-$C_4$-Alkylsulfonyl substituiert ist], für $C_3$-$C_6$-Alkenyloxy [welches gegebenen falls durch Fluor, Chlor, Brom, Cyano oder $C_1$-$C_4$-Alkoxy-carbonyl substituiert ist], für $C_2$-$C_6$-Alkenylthio [welches gegebenenfalls durch Fluor, Chlor, Brom, Cyano, Nitro, $C_1$-$C_3$-Alkylthio oder $C_1$-$C_4$-Alkoxycarbonyl substituiert ist], $C_3$-$C_6$-Alkinyloxy, $C_3$-$C_6$-Alkinylthio, oder für den Rest $-S(O)_p-R^{11}$ stehen, wobei

p für die Zahlen 1 oder 2 steht und

$R^{11}$ für $C_1$-$C_4$-Alkyl [welches gegebenenfalls durch Fluor, Chlor, Brom, Cyano oder $C_1$-$C_4$-Alkoxycarbonyl substituiert ist], $C_3$-$C_6$-Alkenyl, $C_3$-$C_6$-Alkinyl, $C_1$-$C_4$-Alkoxy, $C_1$-$C_4$-Alkoxy-$C_1$-$C_4$-alkylamino, $C_1$-$C_4$-Alkylamino, Di-($C_1$-$C_4$-alkyl)-amino oder für den Rest $-NHOR^{12}$ steht, wobei

$R^{12}$ für $C_1$-$C_{12}$-Alkyl [welches gegebenenfalls durch Fluor, Chlor, Cyano, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkyl-carbonyl, $C_1$-$C_4$- Alkoxy-carbonyl, $C_1$-$C_4$-Alkylamino-carbonyl oder Di-($C_1$-$C_4$-alkyl)-amino-carbonyl substituiert ist], für $C_3$-$C_6$-Alkenyl [welches gegebenenfalls durch Fluor, Chlor oder Brom substituiert ist], $C_3$-$C_6$-Alkinyl, $C_3$-$C_6$-Cycloalkyl, $C_3$-$C_6$-Cycloalkyl-$C_1$-$C_2$-alkyl, Phenyl-$C_1$-$C_2$-alkyl [welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist], für Benzylhydryl oder für Phenyl [welches gegebenenfalls durch Fluor, Chlor, Nitro, Cyano, $C_1$-$C_4$-Alkyl, Trifluormethyl, $C_1$-$C_4$-Alkoxy, $C_1$-$C_2$-Fluoralkoxy, $C_1$-$C_4$-Alkylthio, Trifluormethylthio oder $C_1$-$C_4$-Alkoxy-carbonyl substituiert ist] steht,

$R^9$ und $R^{10}$ weiterhin für Phenyl oder Phenoxy, für Amino, $C_1$-$C_4$-Alkylcarbonylamino, $C_1$-$C_4$-Alkoxy-carbonylamino, $C_1$-$C_4$-Alkylamino-carbonyl-amino, Di-($C_1$-$C_4$-alkyl)-amino-carbonylamino, oder für den Rest $-CO-R^{13}$ stehen, wobei

$R^{13}$ für $C_1$-$C_6$-Alkyl, $C_1$-$C_6$-Alkoxy, $C_3$-$C_6$-Cycloalkoxy, $C_3$-$C_6$-Alkenyloxy, $C_1$-$C_4$-Alkylthio, $C_1$-$C_4$-Alkylamino, $C_1$-$C_4$-Alkoxyamino, $C_1$-$C_4$-Alkoxy-$C_1$-$C_4$-alkylamino oder Di-($C_1$-$C_4$-alkyl)-amino steht [welche gegebenenfalls durch Fluor und/oder Chlor substituiert sind],

$R^9$ und $R^{10}$ weiterhin für $C_1$-$C_4$-Alkylsulfonyloxy, Di-($C_1$-$C_4$-alkyl)-aminosulfonylamino, Thiazolyloxy oder für den Rest $-CH=N-R^{14}$ stehen, wobei

$R^{14}$ für gegebenenfalls durch Fluor, Chlor, Cyano, Carboxy, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes $C_1$-$C_6$-Alkyl, für gegebenenfalls durch Fluor oder Chlor substituiertes Benzyl, für gegebenenfalls durch Fluor oder Chlor substituiertes $C_3$-$C_6$-Alkenyl oder $C_3$-$C_6$-Alkinyl, für gegebenenfalls durch Fluor, Chlor, Brom, $C_1$-$C_4$-Alkyl, $C_1$-$C_4$-Alkoxy, Trifluormethyl, Trifluormethoxy oder Trifluormethylthio substituiertes Phenyl, für gegebenenfalls durch Fluor und/oder Chlor substituiertes $C_1$-$C_6$-Alkoxy, $C_3$-$C_6$-Alkenoxy, $C_3$-$C_6$- Alkinoxy oder Benzyloxy, für Amino, $C_1$-$C_4$-Alkylamino, Di-($C_1$-$C_4$-alkyl)-amino, Phenylamino, $C_1$-$C_4$-Alkyl-carbonyl-amino, $C_1$-$C_4$-Alkoxy-carbonylamino, $C_1$-$C_4$-Alkylsulfonylamino oder für gegebenenfalls durch Fluor, Chlor, Brom oder Methyl substituiertes Phenylsulfonylamino steht,

weiter

$R^1$ für den Rest

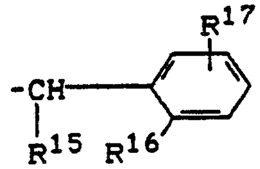

steht, worin

$R^{15}$ für Wasserstoff oder $C_1$-$C_4$-Alkyl steht,

$R^{16}$ und $R^{17}$ gleich oder verschieden sind und für Wasserstoff, Fluor, Chlor, Brom, Nitro, Cyano, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], Carboxy, $C_1$-$C_4$-Alkoxy-carbonyl, $C_1$-$C_4$-Alkylsulfonyl oder Di-($C_1$-$C_4$-alkyl)-aminosulfonyl stehen; weiter

$R^1$ für den Rest

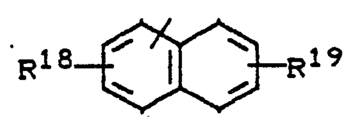

steht, worin
$R^{18}$ und $R^{19}$ gleich oder verschieden sind und für Wasserstoff, Fluor, Chlor, Brom, Nitro, Cyano, $C_1$-$C_4$-Alkyl [welches gegebenenfalls durch Fluor und/oder Chlor substituiert ist] oder $C_1$-$C_4$Alkoxy [welches gegebenenfalls durch Fluor und/oder Chlor substituiert ist], stehen; weiter
$R^1$ für den Rest

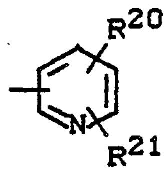

steht, worin
$R^{20}$ und $R^{21}$ gleich oder verschieden sind und für Wasserstoff, Fluor, Chlor, Brom, Nitro, Cyano, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], für $C_1$-$C_4$-Alkylthio, $C_1$-$C_4$-Alkylsulfinyl oder $C_1$-$C_4$-Alkylsulfonyl [welche gegebenenfalls durch Fluor und/oder Chlor substituiert sind], sowie für Di-($C_1$-$C_4$-alkyl)-aminosulfonyl oder $C_1$-$C_4$-Alkoxy-carbonyl stehen; weiter
$R^1$ für den Rest

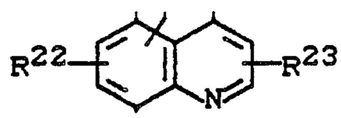

steht, worin
$R^{22}$ und $R^{23}$ gleich oder verschieden sind und für Wasserstoff, Fluor, Chlor, Brom, $C_1$-$C_4$-Alkyl [welches gegebenenfalls durch Fluor und/oder Brom substituiert ist], $C_1$-$C_4$-Alkoxy [welches gegebenenfalls durch Fluor und/ oder Chlor substituiert ist], für $C_1$-$C_4$-Alkylthio, $C_1$-$C_4$-Alkylsulfinyl oder $C_1$-$C_4$-Alkylsulfonyl [welche gegebenenfalls durch Fluor und/oder Chlor substituiert sind], oder für Di-($C_1$-$C_4$-alkyl)-aminosulfonyl stehen; weiter
$R^1$ für den Rest

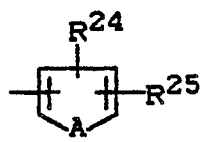

steht, worin
$R^{24}$ und $R^{25}$ gleich oder verschieden sind und für Wasserstoff, Fluor, Chlor, Brom, Cyano, Nitro, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], $C_1$-$C_4$-Alkylthio, $C_1$-$C_4$-Alkylsulfinyl oder $C_1$-$C_4$-Alkylsulfonyl [welches gegebenenfalls durch Fluor und/oder Chlor substituiert ist], Di-($C_1$-$C_4$-alkyl)-aminosulfonyl oder $C_1$-$C_4$-Alkoxy-carbonyl stehen, und
A für Sauerstoff, Schwefel oder die Gruppierung N-$Z^1$ steht, wobei
$Z^1$ für Wasserstoff, $C_1$-$C_4$-Alkyl [welches gegebenenfalls durch Fluor, Chlor, Brom oder Cyano substituiert ist], $C_3$-$C_6$-Cycloalkyl, Benzyl, Phenyl [welches gegebenenfalls durch Fluor, Chlor, Brom oder Nitro substituiert ist], $C_1$-$C_4$-Alkylcarbonyl, $C_1$-$C_4$-Alkoxy-carbonyl oder Di-($C_1$-$C_4$-alkyl)-aminocarbonyl steht; weiter
$R^1$ für den Rest

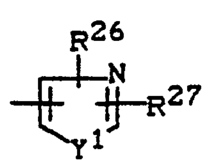

steht, worin
$R^{26}$ für Wasserstoff, $C_1$-$C_5$-Alkyl oder Halogen steht,
$R^{27}$ für Wasserstoff oder $C_1$-$C_5$-Alkyl steht und
$Y^1$ für Schwefel oder die Gruppierung N-$R^{28}$ steht, wobei
$R^{28}$ für Wasserstoff oder $C_1$-$C_5$-Alkyl steht,
weiter
$R^1$ für den Rest

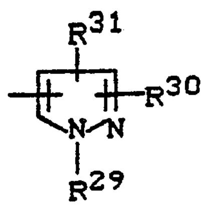

steht, worin
$R^{29}$ für Wasserstoff, $C_1$-$C_4$-Alkyl oder Phenyl steht,
$R^{30}$ für Wasserstoff, Halogen, Cyano, Nitro, $C_1$-$C_4$-Alkyl [welches gegebenenfalls durch Fluor und/ oder Chlor substituiert ist], $C_1$-$C_4$-Alkoxy [welches gegebenenfalls durch Fluor und/oder Chlor substituiert ist] oder $C_1$-$C_4$-Alkoxy-carbonyl steht und
$R^{31}$ für Wasserstoff, Halogen oder $C_1$-$C_4$-Alkyl steht,
weiter
$R^1$ für den Rest

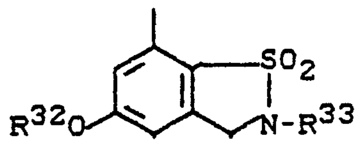

steht, worin
$R^{32}$ für $C_1$-$C_3$-Alkyl steht und
$R^{33}$ für $C_1$-$C_4$-Alkyl steht,
weiter
$R^1$ für den Rest

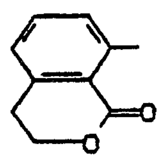

steht,
weiter
$R^2$ die oben für $R^1$ vorzugsweise angegebene Bedeutung hat, jedoch nicht in jedem Einzelfall mit $R^1$ identisch sein muß, und weiter für Di-($C_1$-$C_3$-alkyl)-amino oder für gegebenenfalls durch Halogen substituiertes $C_1$-$C_8$-Alkyl stehen kann,
weiter
$R^3$ für Wasserstoff, Fluor, Chlor, Brom, $C_1$-$C_4$-Alkyl, $C_1$-$C_4$-Halogenalkyl, $C_1$-$C_2$-Alkoxy-$C_1$-$C_2$-alkyl, $C_1$-$C_4$-Alkoxy, $C_1$-$C_4$-Halogenalkoxy, $C_1$-$C_4$-Alkylthio, $C_1$-$C_4$-Halogenalkylthio, Amino, $C_1$-$C_4$-Alkylamino, Dimethylamino oder Diethylamino steht,
X für Stickstoff oder eine -CH-Gruppierung steht,
Y für Stickstoff oder eine -$CR^4$-Gruppierung steht, worin
$R^4$ für Wasserstoff, Fluor, Chlor, Brom, Cyano, Methyl, Formyl, Acetyl, Methoxycarbonyl oder Ethoxycarbonyl steht, und
Z für Stickstoff oder eine -$CR^5$-Gruppierung steht, worin

$R^5$ für Wasserstoff, Fluor, Chlor, Brom, $C_1$-$C_4$-Alkyl, $C_1$-$C_4$-Alkoxy, $C_1$-$C_4$-Halogenalkoxy, $C_1$-$C_4$-Alkylthio, $C_1$-$C_4$-Alkylamino, Dimethylamino oder Diethylamino steht, und
M für Wasserstoff oder ein Natrium-, Kalium-oder Calciumäquivalent, ein Ammonium-, ein $C_1$-$C_6$-Alkylammonium-, ein Di-($C_1$-$C_4$-alkyl)-ammonium oder ein Tri-($C_1$-$C_4$-alkyl)-ammonium-äquivalent steht, wobei N′-(4,6-Dimethyl-pyrimidin-2-yl)-N″-(4-methyl-phenylsulfonylamino)-N‴-(2-chlor-phenylsulfonyl)-guanidin ausgenommen ist.

3. Sulfonylaminoguanidinoazine der allgemeinen Formel (I) gemäß Anspruch 1, dadurch gekennzeichnet, daß darin
$R^1$ für den Rest

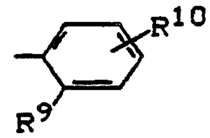

steht, worin
$R^9$ für Fluor, Chlor, Brom, Methyl, Trifluormethyl, Methoxy, Difluormethoxy, Trifluormethoxy, $C_1$-$C_3$-Alkylthio, $C_1$-$C_3$-Alkylsulfinyl, $C_1$-$C_3$-Alkylsulfonyl, Dimethylaminosulfonyl, Diethylaminosulfonyl, N-Methoxy-N-methylaminosulfonyl, Phenyl, Phenoxy oder $C_1$-$C_3$-Alkoxy-carbonyl steht und
$R^{10}$ für Wasserstoff steht; weiter
$R^1$ für den Rest

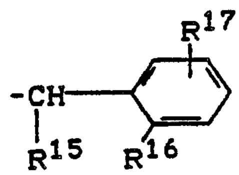

steht, worin
$R^{15}$ für Wasserstoff steht
$R^{16}$ für Fluor, Chlor, Brom, Methyl, Methoxy, Ethoxy, Difluormethoxy, Trifluormethoxy, Methoxycarbonyl, Ethoxycarbonyl, Methylsulfonyl oder Dimethylaminosulfonyl steht und
$R^{17}$ für Wasserstoff steht; weiter
$R^1$ für den Rest

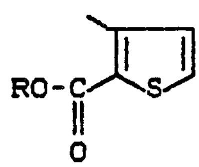

steht, worin
R für $C_1$-$C_2$-Alkyl steht, oder
$R^1$ für den Rest

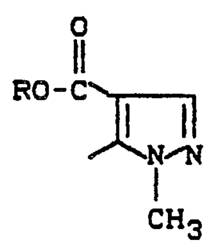

steht, worin

R für $C_1$-$C_2$-Alkyl steht;
weiter
$R^2$ für gegebenenfalls durch Fluor oder Chlor substituiertes $C_1$-$C_4$-Alkyl oder für gegebenenfalls durch Fluor, Chlor, Brom, $C_1$-$C_3$-Alkyl, Trifluormethyl, Chlordifluormethyl, Methoxy, Ethoxy, Difluormethoxy, Trifluormethoxy, Amino, Acetylamino, Methoxycarbonyl und/oder Ethoxycarbonyl substituiertes Phenyl steht,
$R^3$ für Wasserstoff, Fluor, Chlor, Brom, Methyl, Trifluormethyl, Methoxy, Ethoxy, Difluormethoxy, Methylthio, Ethylthio, Amino, Methylamino, Ethylamino, Dimethylamino oder Diethylamino steht,
X für Stickstoff oder eine -CH-Gruppierung steht,
Y für Stickstoff oder eine -$CR^4$-Gruppierung steht, worin
$R^4$ für Wasserstoff, Fluor, Chlor oder Methyl steht,
Z für Stickstoff oder eine -$CR^5$-Gruppierung steht, worin
$R^5$ für Wasserstoff, Fluor, Chlor, Brom, Methyl, Ethyl, Methoxy, Ethoxy, Propoxy, Isopropoxy, Difluormethoxy, Methylthio, Ethylthio, Methylamino, Ethylamino, Dimethylamino oder Diethylamino steht, und
M für Wasserstoff oder ein Natrium-, Kalium-oder Calciumäquivalent, ein Ammonium- oder ein $C_1$-$C_4$-Alkylammoniumäquivalent steht,
wobei N′-(4,6-Dimethyl-pyrimidin-2-yl)-N″-(4-methyl-phenylsulfonylamino)-N‴-(2-chlor-phenylsulfonyl)-guanidin ausgenommen ist.

4. Verfahren zur Herstellung von Sulfonylaminoguanidinoazinen der allgemeinen Formel (I), gemäß Anspruch 1, dadurch gekennzeichnet, daß man Sulfonylverbindungen der allgemeinen Formel (II)

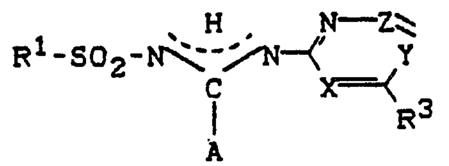

(II)

in welcher
$R^1$, $R^3$, X, Y und Z die in Anspruch 1 angegebenen Bedeutungen haben und
A für eine der nachstehend angegebenen Abgangsgruppen $R^6$-$SO_2$-N(-)-$OR^7$
oder -Q-$R^8$ steht, worin
$R^6$ die in Anspruch 1 für $R^1$ angegebene Bedeutung hat, aber nicht in jedem Einzelfall mit $R^1$ identisch sein muß,
$R^7$ für Alkyl, Alkenyl oder Aralkyl steht,
$R^8$ für Alkyl, Aralkyl oder Aryl steht und
Q für Sauerstoff oder Schwefel steht,
mit Sulfonsäurehydraziden der allgemeinen Formel (III)
$R^2$ - $SO_2$ - NH - $NH_2$ (III)
in welcher
$R^2$ die in Anspruch 1 angegebene Bedeutung hat,
gegebenenfalls in Gegenwart eines Verdünnungsmittels umsetzt und gegebenenfalls die so erhaltenen Produkte nach üblichen Methoden in Salze überführt.

5. Herbizide Mittel, gekennzeichnet durch einen Gehalt an Sulfonylaminoguanidinoazinen der Formel (I) gemäß Anspruch 1.

6. Verfahren zur Bekämpfung von unerwünschtem Pflanzenwachstum, dadurch gekennzeichnet, daß man Sulfonylaminoguanidinoazine der Formel (I) gemäß Anspruch 1 auf die unerwünschten Pflanzen oder ihren Lebensraum einwirken läßt.

7. Verwendung von Sulfonylaminoguanidinoazinen der Formel (I) gemäß Anspruch 1 zur Bekämpfung von Pflanzenwachstum.

8. Verfahren zur Herstellung von herbiziden Mitteln, dadurch gekennzeichnet, daß man Sulfonylaminoguanidinoazine der Formel (I) gemäß Anspruch 1 mit Streckmitteln und/oder oberflächenaktiven Mitteln vermischt.